# FASCICULE DE BREVET EUROPEEN

(11) **EP 2 999 703 B1**
(45) Date de publication et mention de la délivrance du brevet: **24.10.2018**
(21) Numéro de dépôt: 14733226.6
(22) Date de dépôt: 21.05.2014
(51) Int. Cl.: C07D 491/056, C07D 495/04, B32B 5/14, C09D 5/08, C25D 13/20, C09D 5/44, C25D 13/22, B32B 15/08, B32B 15/18, B32B 15/20, C09D 5/16

(54) **MATERIAU SUPEROLEOPHOBE ET/OU SUPERHYDROPHOBE, SON PROCEDE DE PREPARATION ET SES APPLICATIONS**
EXTREM OLEOPHOBES UND/ODER HYDROPHOBES MATERIAL, VERFAHREN ZUR HERSTELLUNG DAVON UND ANWENDUNGEN DAVON
SUPEROLEOPHOBIC AND/OR SUPERHYDROPHOBIC MATERIAL, PROCESS FOR PREPARING SAME AND APPLICATIONS THEREOF

(30) Priorité: 23.05.2013 FR 1301175
(43) Date de publication de la demande: 30.03.2016
(73) Titulaire: CNRS (Centre National de la Recherche Scientifique), 75016 Paris Cedex 16 (FR); Universite De Nice-Sophia Antipolis, 06100 Nice (FR)
(72) Inventeur: CELIA, Elena, F-06800 Cagnes sur Mer (FR); TARRADE, Jeanne, Hélène, Suzanne, F-06400 Cannes (FR); DARMANIN, Thierry, F-06000 Nice (FR); GUITTARD, Frédéric, F-06000 Nice (FR)
(74) Mandataire: Macquet, Christophe
(86) Numéro de dépôt international: PCT/FR2014/000113
(87) Numéro de publication internationale: WO 2014/188089

(56) Documents cités:
- THIERRY DARMANIN ET AL: "Hydrocarbon versus Fluorocarbon in the Electrodeposition of Superhydrophobic Polymer Films", LANGMUIR, vol. 26, no. 22, 16 novembre 2010 (2010-11-16), pages 17596-17602, XP055107996, ISSN: 0743-7463, DOI: 10.1021/la103310m
- JEANNE TARRADE ET AL: "Super liquid-repellent properties of electrodeposited hydrocarbon and fluorocarbon copolymers", RSC ADVANCES, vol. 3, no. 27, 8 mai 2013 (2013-05-08), page 10848, XP055107887, ISSN: 2046-2069, DOI: 10.1039/c3ra40636a
- MÉLANIE WOLFS ET AL: "Versatile Superhydrophobic Surfaces from a Bioinspired Approach", MACROMOLECULES, vol. 44, no. 23, 13 décembre 2011 (2011-12-13), pages 9286-9294, XP055099516, ISSN: 0024-9297, DOI: 10.1021/ma200430n
- YU-CHUAN LIU ET AL: "Effect of Argon Plasma Treatment on Surface-Enhanced Raman Spectroscopy of Polypyrrole Deposited on Electrochemically Roughened Gold Substrates", THE JOURNAL OF PHYSICAL CHEMISTRY B, vol. 109, no. 12, mars 2005 (2005-03), pages 5779-5782, XP055107567, ISSN: 1520-6106, DOI: 10.1021/jp045313l
- TUKEN T ET AL: "The use of polythiophene for mild steel protection", PROGRESS IN ORGANIC COATINGS, ELSEVIER BV, NL, vol. 51, no. 3, décembre 2004 (2004-12), pages 205-212, XP004610268, ISSN: 0300-9440, DOI: 10.1016/J.PORGCOAT.2004.07.013
- GALIT SHUSTAK ET AL: "n -Alkanoic Acid Monolayers on 316L Stainless Steel Promote the Adhesion of Electropolymerized Polypyrrole Films", LANGMUIR, vol. 22, no. 12, juin 2006 (2006-06), pages 5237-5240, XP055107530, ISSN: 0743-7463, DOI: 10.1021/la060460r
- SONIA OBEROI ET AL: "Synthesis of Specially Designed Adhesion Promoter for Grafting Polypyrrole", MACROMOLECULAR SYMPOSIA, vol. 254, no. 1, août 2007 (2007-08), pages 284-291, XP055108037, ISSN: 1022-1360, DOI: 10.1002/masy.200750842
- SHUWEI YANG ET AL: "Modification of ITO Surface for High Performance of Electrochromic Polymer Film", ACTA CHIMICA SINICA, vol. 71, no. 07, 2 mai 2013 (2013-05-02), pages 1041-1046, XP055107614, ISSN: 0567-7351, DOI: 10.6023/A13020168

## Description

La présente invention concerne l'élaboration d'un matériau superoléophobe et/ou superhydrophobe. Plus particulièrement, l'invention concerne un matériau multicouche superoléophobe et/ou superhydrophobe comprenant d'une part un premier constituant qui est un substrat conducteur ou préalablement rendu conducteur et d'autre part au moins un autre constituant qui est une couche polymérique ou copolymérique superoléophobe et/ou superhydrophobe composée d'un ou plusieurs monomères à base de cycle aromatique ou hétéroaromatique substitués par une ou plusieurs chaines fluorocarbonées et/ou hydrocarbonées. Elle concerne également un procédé de préparation dudit matériau et ses applications comme matériau anticorrosion, anti-adhésion et/ou antifriction.

Un matériau est défini comme étant superoléophobe ou superhydrophobe lorsqu'il présente des propriétés antiadhésives de haut niveau. Des matériaux superhydrophobes sont caractérisés par le fait qu'un liquide sonde, l'eau, déposé formera un angle de contact ou angle de raccordement au moins supérieur à 140° voire supérieur à 150°. Parmi les surfaces superhydrophobes on trouve des surfaces glissantes (faible hystérèse) ou non glissantes (forte hystérèse). La feuille de lotus illustre parfaitement ce qu'est une surface superhydrophobe glissante. En effet, sur les feuilles de lotus, qui présentent à la fois une double échelle de structuration micro-nanométrique et la présence de matériaux intrinsèquement hydrophobes (des cires) à l'extrême surface, l'eau ruisselle sans les mouiller. Ce phénomène a d'ailleurs été baptisé « effet lotus ». Des matériaux superoléophobes sont caractérisées par le fait qu'un liquide sonde, de type huile, déposé peut former un angle de contact ou angle de raccordement au moins supérieur à 130°, selon le type d'huile utilisé. En effet, différentes huiles peuvent être utilisées pour caractériser la superoléophobie et dans notre cas on choisira des huiles ou des alcanes dans la tension de surface (γ_{LV}) est inférieure ou égale à 35 mN/m. A titre non limitatif d'exemples d'huile on peut citer l'huile de tournesol, l'huile de colza, l'huile de moteur. A titre non limitatif d'exemples d'alcane on peut citer l'hexadécane, le dodécane, le décane, l'octane.

On connait des documents intitulés « Connecter ability to design superhydrophobic and oleophobic surfaces from conducting polymers », Langmuir, 26(16):13545-9, Zenerino A, Darmanin T, Taffin de Givenchy E, Amigoni S, Guittard F et "Hydrocarbon versus Fluorocarbon in the Electrodeposition of Superhydrophobic Polymer Films", Langmuir 2010 26 (22), 17596-17602, Darmanin T, Taffin de Givenchy E, Amigoni S, Guittard F des méthodes de fabrication de surfaces superoléophobes en utilisant des monomères contenant des chaines perfluorées.

La demande EP2210921 décrit également des articles comprenant des revêtements superhydrophobes. La substance hydrophobe est faite de polymères composés de monomères tels que par exemple l'éthylène, le propylène, le styrène ou le tetrafluoroéthylène.

La demande EP2118189 décrit des compositions super ou ultra-hydrophobes comprenant des polymères ayant un angle de raccordement ou angle de contact de l'eau (« *Water Contact* Angle » ou WCA) compris entre au moins 120° et 150° ou plus.

La demande US2008015298 décrit des revêtements superhydrophobes faits de polymères fluorés ayant des unités structurelles de formule -CR¹R²-CFX- et des articles recouverts de tels revêtements, ainsi que les applications possibles associées.

La demande internationale WO2007102960 décrit des compositions autonettoyantes hydrophobes sous forme de films, et ses applications.

Les demandes WO2012075294 et WO2012009238 décrivent des matériaux superhydrophobes composés de polymères ou copolymères à base de cycle aromatique substitué.

Enfin, la demande EP2092026 divulgue des revêtements ultra-hydrophobes à base de copolymères organosilicones.

Toutefois, les revêtements ou matériaux superoléophobes ou superhydrophobes connus à ce jour sont instables, c'est-à-dire qu'ils adhèrent mal à leur substrat. Leurs propriétés superoléophobes ou superhydrophobes sont donc relativement éphémères. Les revêtements doivent donc être remplacés fréquemment, ce qui entraine notamment une pollution liée aux déchets engendrés, ce qui nécessite une maintenance soutenue et qui engendre des coûts importants.

En outre, les revêtements ou matériaux superoléophobes ou superhydrophobes tels que décrits dans les documents ci-dessus sont aussi relativement complexes à élaborer, c'est-à-dire qu'ils nécessitent plusieurs étapes de fabrication. Ceci les rend donc relativement coûteux à mettre en oeuvre. De même, leur élaboration difficile nécessite généralement l'utilisation de produits, de monomères, de polymères notamment fluorés et contenant de longueurs chaînes perfluorés, qui sont fortement toxiques, notamment en termes de bioaccumulation, d'écotoxicité environnementale.

Il existe donc un besoin d'obtenir des matériaux superoléophobes ou superhydrophobes qui présentent une stabilité améliorée, c'est-à-dire qui présentent un pouvoir d'adhésion important à leur substrat, dont le procédé de fabrication soit avantageusement peu onéreux et préférentiellement réalisable en une seule étape selon certains modes de réalisation, avec avantageusement une approche écotoxique favorable.

Aussi, un but que s'est fixé la présente invention est de fournir des matériaux superoléophobes ou superhydrophobes présentant des propriétés anti-adhérentes, antifriction et/ou anticorrosion, qui soient stables, c'est-à-dire dont les couches polymériques ou copolymériques adhérent à leur substrat.

En outre, les matériaux selon l'invention peuvent avantageusement être utilisés pour former des surfaces biomimétiques ou bioniques, c'est-à-dire reproduisant artificiellement des propriétés essentielles d'un ou plusieurs processus ou surfaces biologiques.

Enfin, les matériaux selon l'invention ou les surfaces comprenant ces matériaux sont élaborés par une méthode peu onéreuse et en une seule étape. Ainsi, cette technologie peut être appliquée notamment dans le domaine de l'anti-bioadhésion, permettant d'éviter l'utilisation de biocide sur les surfaces et ainsi concevoir de nouveaux matériaux hautement performant avec une approche écotoxique favorable.
L'invention a donc pour premier objet un matériau superoléophobe et/ou superhydrophobe multicouche comprenant :
d'une part un premier constituant qui est un substrat conducteur ou préalablement rendu conducteur :
   - dont la surface est modifiée par traitement chimique et/ou physique et intégrant une première couche conductrice promoteur d'adhésion; ou
   - intégrant une première couche conductrice promoteur d'adhésion ;
et d'autre part au moins un autre constituant qui est une couche polymérique ou copolymérique superoléophobe et/ou superhydrophobe composée d'un ou plusieurs monomères à base de cycle aromatique ou hétéroaromatique substitués par une ou plusieurs chaines fluorocarbonées et/ou hydrocarbonées,
caractérisé en ce que les différents constituants dudit matériau respectent un gradient croissant d'hydrophobie entre la première couche déposée sur le substrat conducteur ou préalablement rendu conducteur et la dernière couche dudit matériau.
Elle a pour deuxième objet un procédé de fabrication d'un matériau superoléophobe et/ou superhydrophobe selon l'invention comprenant les étapes de :
- traitement physique et/ou chimique 2 d'un substrat conducteur ou préalablement rendu conducteur 1 et dépôt d'une couche conductrice promoteur d'adhésion 3 sur ledit substrat ; ou dépôt d'une couche conductrice promoteur d'adhésion 3 sur ledit substrat ;
- dépôt sur la couche conductrice promoteur d'adhésion 3 d'au moins une couche polymérique ou copolymérique superoléophobe et/ou superhydrophobe 4, 5 ou 6 composée d'un ou plusieurs monomères à base de cycle aromatique ou hétéroaromatique substitués par une ou plusieurs chaines fluorocarbonées et/ou hydrocarbonées ; et de
- récupération du matériau superoléophobe et/ou superhydrophobe.

Enfin, elle a pour troisième objet l'utilisation d'un matériau selon l'invention à titre de matériau anti-adhésion, antifriction et/ou anticorrosion.

L'invention sera mieux comprise à la lecture de la description non limitative qui suit, rédigée au regard des dessins annexés, dans lesquels :
- la figure 1 illustre sur sa partie supérieure, l'absence d'adhésion d'un revêtement superhydrophobe dépourvu de promoteur d'adhésion. Sur la partie inférieure de la figure 1, on peut observer que le revêtement superhydrophobe se retrouve sur le scotch et non pas sur le support ;
- la figure 2 représente un cliché photographique de l'adhésion d'un revêtement promoteur d'adhésion qu'est le polypyrrole ;
- la figure 3 représente un cliché photographique de l'adhésion d'un revêtement superhydrophobe avec un promoteur d'adhésion qu'est le polypyrrole sur de l'acier inoxydable 316 ;
- les figures 4 à 9 schématisent des exemples de réalisations de matériaux selon l'invention ;
- la figure 10 représente des clichés, numérotés 10(a) à 10(f), pris au microscope électronique à balayage (MEB) avec un grossissement de x10 000 et x 25 000 de (a, b) PEDOT-NH-F4, (c, d) PEDOT-NH-F6 et (e, f) PEDOT-NH-F8. Les encarts aux figures 10 (a), 10(c) et 10(e) représentent des gouttelettes d'hexadécane sur ces surfaces ;
- les figures 11(a), 11(b) et 11(c) sont des représentations schématiques de la partie chimique et de la partie physique des angles de contact et de l'influence de la charge de dépôt (Qs) sur les angles de contact. Ces données sont présentées pour trois polymères et quatre liquides sondes (eau, huile de tournesol, diiodométhane et hexadécane) ;
- la figure 12 représente des Voltamogrammes cycliques de PProDOT-Hn (n = 4, 6 ou 8) dans l'acétonitrile (a) et dans le dichlorométhane (b) ;
- la figure 13 représente des clichés pris au microscope électronique à balayage (MEB) de polymères PProDOT-Hₙ électrodéposés dans du l'acétonitrile. Le cliché (a) concerne le PProDOT-H₄, (b) concerne le PProDOT-H₆ et (c) concerne le PProDOT-H₈.
- la figure 14 représente des clichés pris au microscope électronique à balayage (MEB) de polymères PProDOT-Hₙ électrodéposés dans du dichlorométhane. Le cliché (a) concerne le PProDOT-H₄, (b) concerne le PProDOT-H₆ et (c) concerne le PProDOT-H₈.

Le matériau selon l'invention est un matériau superoléophobe et/ou superhydrophobe comprenant :
d'une part un premier constituant qui est un substrat conducteur ou préalablement rendu conducteur 1 :
   - dont la surface est modifiée par traitement chimique et/ou physique 2 et intégrant une première couche conductrice promoteur d'adhésion 3 ; ou
   - intégrant une première couche conductrice promoteur d'adhésion 3 ;
et d'autre part au moins un autre constituant qui est une couche polymérique ou copolymérique superoléophobe et/ou superhydrophobe 4, 5 ou 6 composée d'un ou plusieurs monomères à base de cycle aromatique ou hétéroaromatique substitués par une ou plusieurs chaines fluorocarbonées et/ou hydrocarbonées.

Le matériau selon l'invention se caractérise en ce que les différents constituants dudit matériau respectent un gradient croissant d'hydrophobie entre la première couche déposée sur le substrat conducteur ou préalablement rendu conducteur 1 et la dernière couche dudit matériau.

A titre non limitatif, le substrat conducteur ou préalablement rendu conducteur 1peut notamment être un acier (inoxydable ou non inoxydable), du platine, de l'or, de l'argent, du fer, de l'oxyde d'indium et d'étain (ITO), du titane, du carbone vitreux, du silicium, de l'aluminium, du cuivre, du zinc, du nickel, du laiton, du bronze.

Afin d'augmenter l'adhésion du matériau selon l'invention, la surface du substrat conducteur ou préalablement rendu conducteur 1 est modifiée :
- par des traitements physiques et/ou chimiques 2, sans apport de matière et par déposition d'une couche conductrice promoteur d'adhésion 3, avec apport de matière, ce qui a pour effet de modifier la chimie de la surface;
   ou
- par déposition d'une couche conductrice promoteur d'adhésion 3.

Le traitement physique de la surface du substrat conducteur ou préalablement rendu conducteur 1 peut notamment être réalisé par gravure au plasma (« *plasma etching* » en anglais). Préférentiellement, le traitement physique est réalisé par plasma oxygène ou plasma argon.

Le traitement chimique de la surface du substrat conducteur ou préalablement rendu conducteur 1 peut notamment être réalisé par polissage, sablage, brossage et/ou par une attaque chimique. Avantageusement, le traitement chimique est réalisé par de l'eau royale appelée également eau régale, *Aqua regia* en latin, qui est un mélange d'acide chlorhydrique et d'acide nitrique concentrés dans une proportion de 2 à 4 volumes d'acide chlorhydrique pour 1 d'acide nitrique.

La couche conductrice promoteur d'adhésion 3 peut être déposée selon plusieurs techniques : monocouche auto-assemblée (« self-assembled monolayer » en anglais, acronyme SAM), plasma, sol-gel, électrofilature (« elecrospinning » en anglais), lithographie, polymérisation radicalaire, d'électrodéposition, couche par couche, trempage (« dip-coating » en anglais), enduction centrifuge (« spin-coating » en anglais).

Avantageusement, la monocouche auto-assemblée SAM comprend de l'acide undecylenique, de l'acide undécanoïque, de l'acide décanoïque, de l'acide linoléique, de l'acide octadécyle phosphonique, du pyrrole undécane thiol, du N-(3,4-dihydroxyphenethyl)thiophene-3-carboxamide, de la dopamine, pris seuls ou en mélange.

Avantageusement, la couche d'électrodéposition est composée d'un ou plusieurs monomères à base de cycle aromatique ou hétéroaromatique éventuellement substitué. De préférence, la couche d'électrodéposition est composée d'un ou plusieurs monomères de pyrrole ou d'aniline, pris seuls ou en mélange.

De préférence, la couche d'électrodéposition est composée de monomères de polypyrrole :

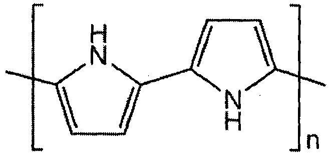

De façon surprenante, le Demandeur a pu mettre en évidence que l'épaisseur de la couche conductrice 3 jouait un rôle primordial sur son adhésion au substrat conducteur ou préalablement rendu conducteur 1. En effet, le Demandeur a établi que la cohésion était inversement proportionnelle à l'épaisseur de la couche déposée. Ainsi, la couche conductrice promoteur d'adhésion a préférentiellement une épaisseur inférieure à environ 100 nm. De préférence encore, l'épaisseur est comprise entre 1 et 50 nm, encore plus préférentiellement 20 nm. Cette épaisseur est fonction de la charge surfacique appliquée qui est préférentiellement comprise entre 1 et 20 mC/cm². De préférence, la charge surfacique est de 5 mC/cm².

Le Demandeur a pu mettre en évidence que, si la couche conductrice 3 est fine, cela évite une rupture cohésive. En outre, il a pu être établi que l'épaisseur du polypyrrole influence les propriétés de mouillabilité du polymère superhydrophobe. Plus l'épaisseur du polypyrrole est importante, plus l'angle de contact du polymère superhydrophobe diminue. Le matériau multicouche selon l'invention comprend en outre au moins un autre constituant qui est une couche polymérique ou copolymérique superoléophobe et/ou superhydrophobe 4, 5 ou 6 composée d'un ou plusieurs monomères à base de cycle aromatique ou hétéroaromatique substitués par une ou plusieurs chaines fluorocarbonées et/ou hydrocarbonées.

De préférence la couche polymérique ou copolymérique superoléophobe et/ou superhydrophobe 4, 5 ou 6 est composée d'un ou plusieurs monomères répondant à la formule générale suivante : 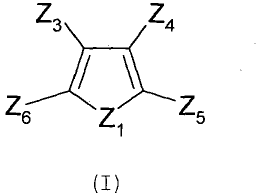 dans laquelle :
- Z₁ est choisi parmi les atomes et groupements S, O, NH et N-W ;
- Z₃ est choisi parmi les atomes et groupements : H, X₁-W, X₁-R₁-X₂, C(R₂)(R₃) ;
- Z₄ est choisi parmi les groupements: W, X₁-W, X₁-R₁-X₂ et C(R₂) (R₃) ;
- Z₅ et Z₆ représentent un atome H ou une chaîne hydrocarbonée comprenant 3 atomes de carbone ;
dans laquelle :
∘ W représente (CF₂)_{q}F, B₁-(CY₂)ₚY, Q₁-Z₂ ou B₁-Q₁-Z₂ ;
   - avec B₁, B₂, B₃ identiques ou différents sont choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
   - avec Z₂ correspondant à A₁, CH(A₁)(A₂),CH₂(A₁), NH(A₁), N(A₁)(A₂), O(A₁) ou S(A₁) ;
      pour lequel A₁ et A₂, identiques ou différents représentent :
      -B₂-(CY₂)ₚY,
      -B₃-ph-Q₂-B₂-(CY₂)ₚY ; « ph » désignant le groupe fonctionnel phényle ;
   - avec q compris entre 1 et 20 et p compris entre 0 et 20 ;
   - avec Y correspondant à H ou F, sous réserve que, lorsque Y est un atome H, alors p est égal à 0 ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S, NH ;
∘ R₁ est choisi parmi les groupements -(CH)W-,-(CH₂)ₙ₁-CH(W)- ; -CH(W)-(CH₂)ₙ₁ ; -(CH₂)ₙ₂-CH(W)-(CH₂)ₙ₃- ; et
   - (CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
- lorsque Z₃ et Z₄ correspondent à X₁-R₁-X₂, ils sont reliés ensemble pour former un cycle ;
- lorsque Z₃ et/ou Z₄ correspondent à C(R₂)(R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
pris seuls ou en mélange.

De préférence encore, la couche polymérique ou copolymérique superoléophobe et/ou superhydrophobe 4, 5 ou 6 selon l'invention est composée d'un ou plusieurs monomères répondant à la formule générale (I) dans laquelle :
- Z₁ est choisi parmi les atomes et groupements S, NH et N-W ;
- Z₃ est choisi parmi les atomes et groupements : H, X₁-W et X₁-R₁-X₂ ;
- Z₄ est choisi parmi les groupements: W, X₁-W et X₁-R₁-X₂ ;
- Z₅ et Z₆ représentent un atome H ;
dans laquelle :
∘ W représente (CF₂)_{q}F, B₁-(CY₂)ₚY, Q₁-Z₂ ou B₁-Q₁-Z₂ ;
   - avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
   - avec Z₂ correspondant à A₁, CH(A₁)(A₂), CH₂(A₁), NH(A₁), N(A₁)(A₂), O(A₁) ou S(A₁) ;
      pour lequel A₁ et A₂, identiques ou différents représentent :
      -B₂-(CY₂)ₚY,
      -B₃-ph-Q₂-B₂-(CY₂)pY ; « ph » désignant le groupe fonctionnel phényle ;
   - avec q compris entre 1 et 16 et p compris entre 0 et 16 ;
   - avec Y correspondant à H ou F, sous réserve que, lorsque Y est un atome H, alors p est égal à 0 ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S ;
∘ R₁ est choisi parmi les groupements-(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
- lorsque Z₃ et Z₄ correspondent à X₁-R₁-X₂, ils sont reliés ensemble pour former un cycle ;
pris seuls ou en mélange.

La couche polymérique ou copolymérique superoléophobe et/ou superhydrophobe 4, 5 ou 6 selon l'invention est préférentiellement composée d'un ou plusieurs monomères choisis parmi :
(i) les composés de formule (I) dans laquelle :
   - Z₁ est choisi parmi les atomes et groupements S, NH et N-W ;
   - Z₃ est choisi parmi les atomes et groupements H, X₁-W et X₁-R₁-X₂ ;
   - Z₄ est choisi parmi les groupements: W, X₁-W et X₁-R₁-X₂ ;
   - Z₅ et Z₆ représentent un atome H ;
   dans laquelle :
   ∘ W représente (CF₂)_{q}F, B₁-(CF₂)ₚF, Q₁-Z₂ ou B₁-Q₁-Z₂ ;
      - avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
      - avec Z₂ correspondant à A₁, CH(A₁)(A₂), CH₂(A₁), NH(A₁), N(A₁)(A₂), O(A₁) ou S(A₁) ; pour lequel A₁ et A₂, identiques ou différents représentent :
         -B₂-(CF₂)ₚF,
         -B₃-ph-Q₂-B₂-(CF₂)ₚF ; « ph » désignant le groupe fonctionnel phényle ;
      - avec q et p compris entre 7 et 16 ;
      - avec Q₁ et Q₂, identiques ou différents qui représentent OC(O), C(O), SC(O), NHC(O) ;
   ∘ X₁ et X₂ ; identiques ou différents, sont choisis parmi les atomes ou groupements O, S;
   ∘ R₁ est choisi parmi les groupements -(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
   et dans laquelle :
   ∘ lorsque Z₃ et Z₄ correspondent à X₁-R₁-X₂, ils sont reliés ensemble pour former un cycle ;
   ou parmi
(ii) les composés sans fluor suivants, répondant également à la formule générale (I) dans laquelle :
   - Z₁ est choisi parmi les atomes et groupements S, O, NH et N-W ;
   - Z₃ est choisi parmi les atomes et groupements H, X₁-W, X₁-R₁-X₂, C(R₂)(R₃) ;
   - Z₄ est choisi parmi les atomes et groupements W, X₁-W, X₁-R₁-X₂, C(R₂)(R₃) ;
   - Z₅ et Z₆ représentent un atome H ou une chaîne hydrocarbonée comprenant 3 atomes de carbone ;
   dans laquelle :
   ∘ W représente B₁-H, Q₁-Z₂ ou B₁-Q₁-Z₂ ;
      - avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
      - avec Z₂ correspondant à A₁, CH(A₁)(A₂), CH₂(A₁), NH(A₁), N(A₁)(A₂), O(A₁) ou S(A₁) ;
         pour lequel A₁ et A₂, identiques ou différents représentent :
         -B₂-H,
         -B₃-ph-Q₂-B₂-H ; « ph » désignant le groupe fonctionnel phényle ;
      - avec Q₁ et Q₂, identiques ou différents qui représentent OC(O), C(O), SC(O), NHC(O) ;
   ∘ X₁ et X₂ ; identiques ou différents, sont choisis parmi les atomes ou groupements O, S, NH ;
   ∘ R₁ est choisi parmi les groupements -(CH)W-, -(CH₂)ₙ₁-CH(W)- ; -CH(W)-(CH₂)ₙ₁ ; -(CH₂)ₙ₂-CH(W)-(CH₂)ₙ₃- ; et
      -(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
   ∘ R₂ est une chaîne hydrocarbonée comprenant 3 atomes de carbone ;
   ∘ R₃ est choisi parmi les groupements W, X₁-W ou X₁-R₁-X₂;
   et dans laquelle :
   - lorsque Z₃ et Z₄ correspondent à X₁-R₁-X₂, ils sont reliés ensemble pour former un cycle ;
   - lorsque Z₃ et/ou Z₄ correspondent à C(R₂)(R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
   ou encore parmi
(iii) les composés à courte chaine fluorée suivants répondant également à la formule générale (I) dans laquelle :
   - Z₁ est choisi parmi les atomes et groupements S, O, NH et N-W ;
   - Z₃ est choisi parmi les atomes et groupements H, X₁-W, X₁-R₁-X₂, C(R₂)(R₃) ;
   - Z₄ est choisi parmi les atomes et groupements W, X₁-W, X₁-R₁-X₂, C(R₂)(R₃) ;
   - Z₅ et Z₆ représentent un atome H ou une chaîne hydrocarbonée comprenant 3 atomes de carbone ;
   dans laquelle :
   ∘ W représente (CF₂)_{q}F, B₁-(CF₂)ₚF, Q₁-Z₂ ou B₁-Q₁₋Z₂ ;
      - avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
      - avec Z₂ correspondant à A₁, CH(A₁)(A₂), CH₂(A₁), NH(A₁), N(A₁)(A₂), O(A₁) ou S(A₁) ;
         pour lequel A₁ et A₂, identiques ou différents représentent :
         -B₂-(CF₂)ₚF,
         -B₃-ph-Q₂-B₂-(CF₂)ₚF ; « ph » désignant le groupe fonctionnel phényle ;
      - avec q et p compris entre 1 et 6;
      - avec Q₁ et Q₂, identiques ou différents qui représentent OC(O), C(O), SC(O), NHC(O) ;
   ∘ X₁ et X₂ ; identiques ou différents, sont choisis parmi les atomes ou groupements O, S, NH ;
   ∘ R₁ est choisi parmi les groupements -(CH)W-, -(CH₂)ₙ₁-CH(W)- ; -CH(W)-(CH₂)ₙ₁ ; -(CH₂)ₙ₂-CH(W)-(CH₂)ₙ₃- ; et
      -(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
   ∘ R₂ est une chaîne hydrocarbonée comprenant 3 atomes de carbone ;
   ∘ R₃ est choisi parmi les groupements W, X₁-W ou X₁-R₁-X₂;
   et dans laquelle :
   - lorsque Z₃ et Z₄ correspondent à X₁-R₁-X₂, ils sont reliés ensemble pour former un cycle ;
   - lorsque Z₃ et/ou Z₄ correspondent à C(R₂) (R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
   pris seuls ou en mélange.

De façon particulièrement avantageuse, la couche polymérique ou copolymérique superoléophobe et/ou superhydrophobe 4, 5 ou 6 selon l'invention est composée d'un ou plusieurs monomères choisis :
parmi les composés sans fluor suivants, répondant à la formule générale (I) dans laquelle :
- Z₁ est choisi parmi les atomes et groupements S, NH et N-W;
- Z₃ est choisi parmi les atomes et groupements H, X₁-W, X₁-R₁-X₂;
- Z₄ est choisi parmi les atomes et groupements W, X₁-W, X₁-R₁-X₂;
- Z₅ et Z₆ représentent un atome H ;
dans laquelle :
∘ W représente B₁-H, Q₁-Z₂ ou B₁-Q₁-Z₂ ;
   - avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
   - avec Z₂ correspondant à A₁, CH(A₁)(A₂),CH₂(A₁), NH(A₁), N(A₁)(A₂), O(A₁) ou S(A₁) ;
      pour lequel A₁ et A₂, identiques ou différents représentent :
      -B₂-H,
      -B₃-ph-Q₂-B₂-H ; « ph » désignant le groupe fonctionnel phényle ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S;
∘ R₁ est choisi parmi les groupements -(CH)W-, -(CH₂)ₙ₁-CH(W)-, -CH(W)-(CH₂)ₙ₁, -(CH₂)ₙ₂-CH(W)-(CH2)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
lorsque Z₃ et Z₄ correspondent à X₁-R₁-X₂, ils sont reliés ensemble pour former un cycle ;
ou parmi les composés à courte chaine fluorée suivants répondant également à la formule générale (I) dans laquelle :
- Z₁ est choisi parmi les atomes et groupements S, NH et N-W ;
- Z₃ est choisi parmi les atomes et groupements H, X₁-W, X₁-R₁-X₂;
- Z₄ est choisi parmi les atomes et groupements W, X₁-W, X₁-R₁-X₂;
- Z₅ et Z₆ représentent un atome H ;
dans laquelle :
∘ W représente (CF₂)₄F, B₁-(CF₂)₄F, Q₁-Z₂ ou B₁-Q₁-Z₂ ;
   - avec B₁, B₂, B₃ identiques ou différents choisis parmi les groupements : C₁-C₁₄ alkyles;
   - avec Z₂ correspondant à A₁, CH(A₁)(A₂),CH₂(A₁), NH(A₁), N(A₁)(A₂), O(A₁) ou S(A₁) ;
      pour lequel A₁ et A₂, identiques ou différents représentent :
      -B₂-(CF₂)₄F,
      -B₃-ph-Q₂-B₂-(CF₂)₄F ; « ph » désignant le groupe fonctionnel phényle ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S ;
∘ R₁ est choisi parmi les groupements -(CH)W-, -(CH₂)ₙ₁-CH(W)-, -CH(W)-(CH₂)ₙ₁, -(CH₂)n₂-CH(W)-(CH₂)ₙ₃-, -(CH₂)ₙ₁ lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
lorsque Z₃ et Z₄ correspondent à X₁-R₁-X₂, ils sont reliés ensemble pour former un cycle;
pris seuls ou en mélange.

Selon un premier mode de réalisation avantageux de l'invention, la couche polymérique ou copolymérique superoléophobe et/ou superhydrophobe 4, 5 ou 6 est composée uniquement d'un ou plusieurs monomères sans fluor répondant à la formule générale (I) dans laquelle :
- Z₁ est choisi parmi les atomes et groupements S, O, NH et N-W ;
- Z₃ est choisi parmi les atomes et groupements H, X₁-W, X₁-R₁-X₂, C(R₂)(R₃) ;
- Z₄ est choisi parmi les atomes et groupements W, X₁-W, X₁-R₁-X₂, C(R₂) (R₃) ;
- Z₅ et Z₆ représentent un atome H ou une chaîne hydrocarbonée comprenant 3 atomes de carbone ;
dans laquelle :
∘ W représente B₁-H, Q₁-Z₂ ou B₁-Q₁-Z₂ ;
   - avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
   - avec Z₂ correspondant à A₁,CH(A₁)(A₂),CH₂(A₁), NH(A₁), N(A₁)(A₂), O(A₁) ou S(A₁) ;
      pour lequel A₁ et A₂, identiques ou différents représentent :
      -B₂-H,
      -B₃-ph-Q₂-B₂-H ; « ph » désignant le groupe fonctionnel phényle ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements 0, S, NH ;
∘ R₁ est choisi parmi les groupements -(CH)W-, -(CH₂)ₙ₁-CH(W)- ; -CH(W)-(CH₂)ₙ₁ ; -(CH₂)ₙ₂-CH(W)-(CH₂)ₙ₃- ; et
   -(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
- lorsque Z₃ et Z₄ correspondent à X₁-R₁-X₂, ils sont reliés ensemble pour former un cycle ;
- lorsque Z₃ et/ou Z₄ correspondent à C(R₂)(R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
pris seuls ou en mélange.

Préférentiellement, selon ce premier mode de réalisation avantageux, la couche polymérique ou copolymérique superoléophobe et/ou superhydrophobe 4, 5 ou 6 est composée uniquement d'un ou plusieurs monomères répondant à la formule (II): 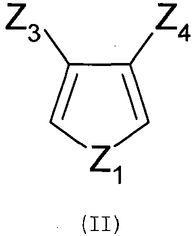 dans laquelle :
- Z₁ est choisi parmi les atomes et groupements S, NH et N-W;
- Z₃ et Z₄ représentent X₁-R₁-X₂ et sont reliés ensemble pour former un cycle,
   ∘ X₁ et X₂ sont choisis parmi les atomes et groupements 0, S;
   ∘ R₁ est choisi parmi les groupements suivants :
      -(CH₂)ₙ₁-CH(W)-,
      -CH(W)-(CH₂)n₁,
      -(CH₂)ₙ₂-CH(W)-(CH₂)ₙ₃- ;
   ∘ W représente -B₁-H, Q₁-Z₂ ou B₁-Q₁-Z₂ où Z₂ correspondant à A₁, CH(A₁)(A₂), CH₂(A₁), NH(A₁), N(A₁)(A₂), O(A₁) ou S(A₁) ;
      pour lequel A₁ et A₂, identiques ou différents représentent :
      -B₂-H, avec B₁, B₂, identiques ou différents choisis parmi les groupements C₁-C₁₆ alkyles, C₁-C₁₄ alkényles, C₁-C₁₆ alkynyles, C₆-C₁₈ aryles, C₆-C₁₈ hétéroaryles ;
   avec Q₁ représentant OC(O), C(O), SC(O), NHC(O) n1, n2 et n3 identiques ou différents, sont compris entre 1 et 4, la somme de n2 + n3 étant inférieure ou égale à 4 ;
pris seuls ou en mélange.
Selon un deuxième mode de réalisation avantageux de l'invention, la couche polymérique ou copolymérique superoléophobe et/ou superhydrophobe 4, 5 ou 6 est composée uniquement d'un ou plusieurs monomères à courte chaine fluorée répondant à la formule générale (I) dans laquelle :
- Z₁ est choisi parmi les atomes et groupements S, O, NH et N-W ;
- Z₃ est choisi parmi les atomes et groupements H, X₁-W, X₁-R₁-X₂, C(R₂)(R₃) ;
- Z₄ est choisi parmi les atomes et groupements W, X₁-W, X₁-R₁-X₂, C(R₂)(R₃) ;
- Z₅ et Z₆ représentent un atome H ou une chaîne hydrocarbonée comprenant 3 atomes de carbone ;
dans laquelle :
∘ W représente (CF₂)_{q}F, B₁-(CF₂)ₚF, Q₁-Z₂ ou B₁-Q₁-Z₂ ;
   - avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
   - avec Z₂ correspondant à A₁, CH(A₁)(A₂), CH₂(A₁), NH(A₁), N(A₁)(A₂), O(A₁) ou S(A₁) ;
      pour lequel A₁ et A₂, identiques ou différents représentent :
      -B₂-(CF₂)ₚF,
      -B₃-ph-Q₂-B₂-(CF₂)ₚF ; « ph » désignant le groupe fonctionnel phényle ;
   - avec q et p compris entre 1 et 6 ;
   - avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S, NH ;
∘ R₁ est choisi parmi les groupements -(CH)W-, -(CH₂)ₙ₁-CH(W)- ; -CH(W)-(CH₂)ₙ₁ ; -(CH₂)ₙ₂-CH(W)-(CH₂)ₙ₃- ; et
   -(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
- lorsque Z₃ et Z₄ correspondent à X₁-R₁-X₂, ils sont reliés ensemble pour former un cycle ;
- lorsque Z₃ et/ou Z₄ correspondent à C(R₂) (R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
pris seuls ou en mélange.

Préférentiellement, selon ce deuxième mode de réalisation avantageux, la couche polymérique ou copolymérique superoléophobe et/ou superhydrophobe 4, 5 ou 6 est composée uniquement d'un ou plusieurs monomères répondant à la formule suivante : 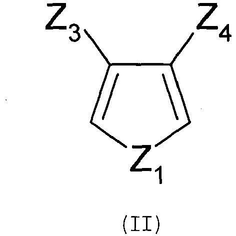 dans laquelle :
- Z₁ est choisi parmi les atomes et groupements S, NH et N-W,
- Z₃ et Z₄ représentent X₁-R₁-X₂ et sont reliés ensemble pour former un cycle ;
   ∘ X₁ et X₂ sont choisis parmi les atomes et groupements 0 et S;
   ∘ R₁ est choisi parmi les groupements suivants :
      -(CH₂)ₙ₁-CH(W)-,
      -CH(W)-(CH₂)ₙ₁,
      -(CH₂)ₙ₂-CH(W)-(CH₂)ₙ₃- ;
   ∘ W représente -B₁-(CF₂)₄F, Q₁-Z₂ ou B₁-Q₁-Z₂ où Z₂ correspondant à A₁, CH(A₁)(A₂), CH₂(A₁), NH(A₁), N(A₁)(A₂), O(A₁) ou S(A₁) ;
      pour lequel A₁ et A₂, identiques ou différents représentent :
      -B₂-(CF₂)₄F, avec B₁, B₂, identiques ou différents choisis parmi les groupements C₁-C₁₄ alkyles;
   avec Q₁ représentant OC(O), C(O), SC(O), NHC(O)
   n1, n2 et n3 identiques ou différents, sont compris entre 1 et 4, la somme de n2 + n3 étant inférieure ou égale à 4 ;
   pris seuls ou en mélange.

A titre d'exemples non limitatifs de monomères utilisables pour la couche polymérique ou copolymérique superoléophobe et/ou superhydrophobe 4, 5 ou 6, on peut citer les composés suivants :
- 2-(2*H*-[1,4]dioxino[2,3-*c*]pyrrol-6(3*H*)-yl)ethyl 4,4,5,5,6,6,7,7,7-nonafluoroheptanoate ;
- 2-(2*H*-[1,4]dithiino[2,3-*c*]pyrrol-6(3*H*)-yl)ethyl 4,4,5,5,6,6,7,7,7-nonafluoroheptanoate ;
- 3-(2*H*-[1,4]dioxino[2,3-*c*]pyrrol-6(3*H*)-yl)propyl 4,4,5,5,6,6,7,7,7-nonafluoroheptanoate ;
- (2*H*-[1,=4]dioxino[2,3-*c*]pyrrol-6(3*H*)-yl)dodecyl 4,4,5,5,6,6,7,7,7-nonafluoroheptanoate ;
- 3,3,4,4,5,5,6,6,6-nonafluorohexyl 2-(1*H*-pyrrol-3-yl)acetate ;
- thiophen-3-ylmethyl 4,4,5,5,6,6,7,7,7-nonafluoroheptanoate ;
- 2-(thiophen-3-yl)ethyl 4,4,5,5,6,6,7,7,7-nonafluoroheptanoate ;
- (2,3-dihydrothieno[3,4-*b*][1,4]dioxin-2-yl)methyl 4,4,5,5,6,6,7,7,7-nonafluoroheptanoate ;
- *S*-((2,3-dihydrothieno[3,4-*b*][1,4]oxathiin-3-yl)methyl) 4,4,5,5,6,6,7,7,7-nonafluoroheptanethioate ;
- (2,3-dihydrothieno[3,4-b][1,4]oxathiin-2-yl)methyl 4,4,5,5,6,6,7,7,7-nonafluoroheptanoate ;
pris seuls ou en mélange.

On peut également citer les composés sans fluor suivants :
- 6-decyl-3,6-dihydro-2*H*-[1,4]dioxino[2,3-*c*]pyrrole ;
- 6-dodecyl-3,6-dihydro-2*H*-[1,4]dioxino[2,3-*c*]pyrrole ;
- 6-tetradecyl-3,6-dihydro-2*H*-[1,4]dioxino[2,3-*c*]pyrrole ;
- 2-octyl-2,3-dihydrothieno[3,4-b][1,4]dioxine ;
- 2-decyl-2,3-dihydrothieno[3,4-b][1,4]dioxine ;
- 2-dodecyl-2,3-dihydrothieno[3,4-b][1,4]dioxine ;
- 2-((octyloxy)methyl)-2,3-dihydrothieno[3,4-b][1,4]dioxine ;
- (2,3-dihydrothieno[3,4-*b*][1,4]dioxin-2-yl)methyl nonanoate ;
- 2-(thiophen-3-yl)ethyl nonanoate ;
- 2-(2*H*-[1,4]dioxino[2,3-*c*]pyrrol-6(3*H*)-yl)ethyl nonanoate ;
- (3,4-dihydro-2*H*-thieno[3,4-*b*][1,4]dioxepin-3-yl)methyl heptanoate ;
- (3,4-dihydro-2*H*-thieno[3,4-*b*][1,4]dioxepin-3-yl)methyl nonanoate ;
- (3,4-dihydro-2*H*-thieno[3,4-*b*][1,4]dioxepin-3-yl)methyl undecanoate ;
- (2,3-dihydrothieno[3,4-b][1,4]dioxin-2-yl)methyl isobutyrate ;
- (2,3-dihydrothieno[3,4-b][1,4]dioxin-2-yl)methyl 2-ethylbutanoate ; (2,3-dihydrothieno[3,4-b][1,4]dioxin-2-yl)methyl 2-propylpentanoate ;
pris seuls ou en mélange.

On peut également citer les composés à courte chaine fluorée suivants:
- 12-(*2H*-[1,4]dioxino[2,3-*c*]pyrrol-6(*3H*)-yl)-*N*-(3,3,4,4,5,5,6,6,6-nonafluorohexyl)dodecanamide ;
- *S*-((2,3-dihydrothieno[3,4-*b*][1,4]oxathiin-2-yl)methyl) 4,4,5,5,6,6,7,7,7-nonafluoroheptanethioate ;
- (3,4-dihydro-2*H*-thieno[3,4-*b*][1,4]dioxepin-3-yl)methyl 4,4,5,5,6,6,7,7,7-nonafluoroheptanoate ;
- 6-(2H-[1,4]dioxino[2,3-*c*]pyrrol-6(3*H*)-yl)hexyl 4,4,5,5,6,6,7,7,7-nonafluoroheptanoate ;
- 1-((2,3-dihydrothieno[3,4-b][1,4]dioxin-2-yl)methyl)-3-(2,2,3,3,4,4,5,5,5-nonafluoropentyl)urea ;
- 2,2,3,3,4,4,5,5,5-nonafluoropentyl ((2,3-dihydrothieno[3,4-*b*][1,4]dioxin-2-yl)methyl)carbamate ;
- (2,3-dihydrothieno[3,4-*b*][1,4]dioxin-2-yl)methyl (2,2,3,3,4,4,5,5,5-nonafluoropentyl)carbamate ;
- 12-((2,3-dihydrothieno[3,4-*b*][1,4]dioxin-2-yl)methoxy)-N-(2,2,3,3,4,4,5,5,6,6,6-undecafluorohexyl)dodecanamide ;
pris seuls ou en mélange.

De façon surprenante, le Demandeur a pu mettre en évidence que le matériau selon l'invention, composé des constituants détaillés ci-dessus, est effectivement superoléophobe et/ou superhydrophobe, tout en présentant à la fois des propriétés anti-adhérentes, antifriction et/ou anticorrosion, mais également une stabilité améliorée, notamment en terme d'adhésion et de cohésion avec le substrat. C'est-à-dire que les couches polymériques ou copolymériques adhérent mieux au substrat dont la surface est modifiée et/ou intègre une première couche conductrice promoteur d'adhésion telle que décrite ci-dessus.

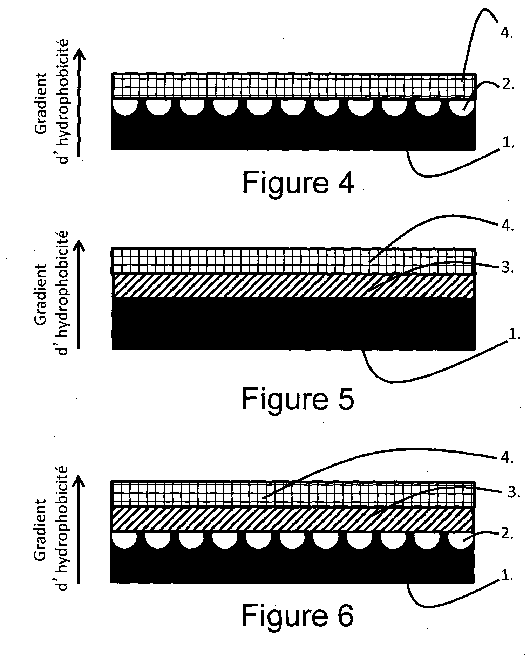

Comme cela est détaillé dans les exemples et illustré aux figures 4 à 9, le Demandeur a mis en évidence que le matériau selon l'invention était particulièrement stable dans le temps du fait de la superposition des différents constituants respectent un gradient croissant d'hydrophobie entre la première couche déposée sur le substrat conducteur ou préalablement rendu conducteur 1 et la dernière couche dudit matériau. Rien dans les documents de l'état de la technique n'enseigne ni ne décrit qu'il soit utile et avantageux d'avoir une superposition de couches polymériques ou copolymériques tels que décrites ci-dessus, avec un gradient d'hydrophobie croissant, afin d'obtenir des matériaux superoléophobes et/ou superhydrophobes présentant des propriétés, notamment d'adhésion, améliorées.

Le Demandeur a pu mettre en évidence qu'il est très difficile voire impossible de déposer la couche superhydrophobe ou superoléophobe selon l'invention directement sur le support, sans traitement de surface préalable ou ajout d'un promoteur.

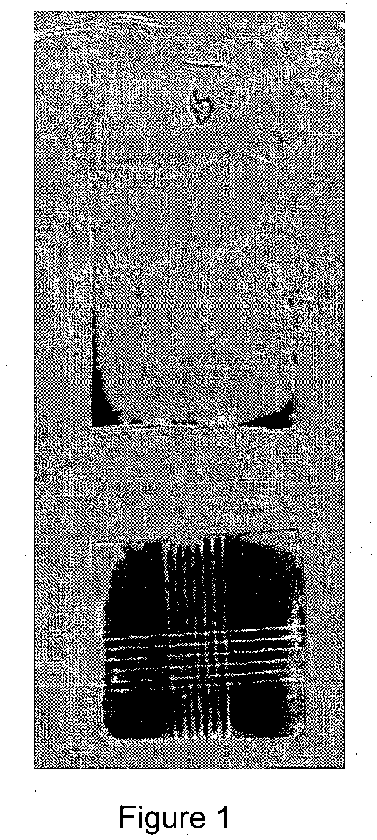

Le Demandeur a pu observer plusieurs phénomènes différents en fonction du type de monomères utilisés. Il a été, par exemple, impossible de déposer des monomères de type EDOTF6 sur un support de type acier. Le dépôt de monomère de type EDOTF4 a permis d'obtenir une surface copolymérique présentant une adhésion très faible (cf. figure 1, résultat d'adhésion 0B selon la méthode ASTM D 3359) .De plus le dépôt n'était pas homogène et il était impossible de retrouver les propriétés intrinsèques des matériaux superoléophobes et/ou superhydrophobes.

De façon avantageuse, le matériau selon l'invention comprend en outre une ou plusieurs couches polymériques ou copolymériques additionnelles 5, 6 qui se superposent par-dessus la première couche polymérique ou copolymérique 4 décrite ci-dessus, selon un gradient croissant d'hydrophobicité.

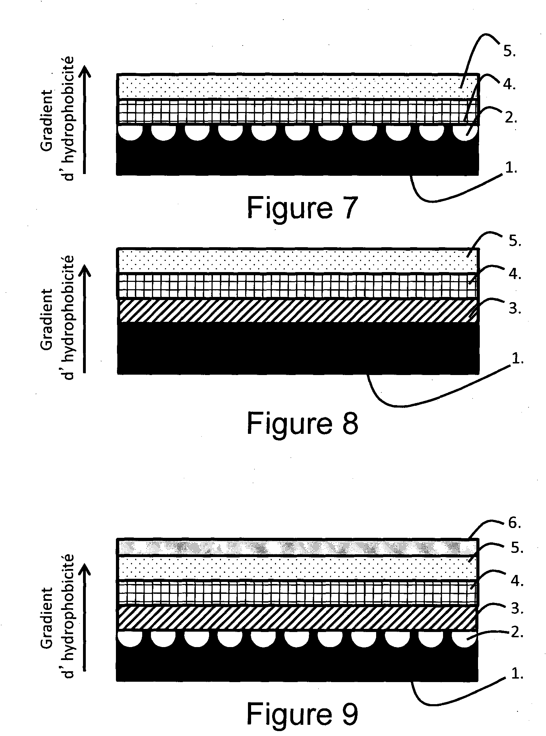

Autrement dit, comme cela est illustré à la figure 9, la couche polymérique ou copolymérique 6 située sur la surface externe du matériau présente une hydrophobicité supérieure aux couches inférieures 3, 4 et 5, éventuellement modifié par traitement chimique ou physique 2.

Un deuxième objet de l'invention concerne un procédé de fabrication d'un matériau superoléophobe et/ou superhydrophobe selon l'invention comprenant les étapes de :
- traitement physique et/ou chimique 2 d'un substrat conducteur ou préalablement rendu conducteur 1 et dépôt d'une couche conductrice promoteur d'adhésion 3 sur ledit substrat ; ou dépôt d'une couche conductrice promoteur d'adhésion 3 sur ledit substrat;
- dépôt sur la couche conductrice promoteur d'adhésion 3 d'au moins une couche polymérique ou copolymérique superoléophobe et/ou superhydrophobe 4, 5 ou 6 composée d'un ou plusieurs monomères à base de cycle aromatique ou hétéroaromatique substitués par une ou plusieurs chaines fluorocarbonées et/ou hydrocarbonées ; et de
- récupération du matériau superoléophobe et/ou superhydrophobe.

De préférence, le substrat est déposé dans un bain conducteur composé d'un liquide ionique ou organique ou d'une émulsion ou microémulsion est de l'acétonitrile anhydrique ou un mélange à base d'eau, contenant un sel ou électrolyte tel que notamment le tetrabutylammonium hexafluorophosphate (Bu4NPF6)

Les traitements physique et/ou chimique, la couche polymérique ou copolymérique sont tels que décrits ci-dessus.

Enfin, un troisième objet de l'invention concerne l'utilisation d'un matériau selon l'invention comme matériau anti-adhésion, antifriction et/ou anticorrosion.

Parmi les applications d'anti-adhésion envisageables selon l'invention, les matériaux selon l'invention peuvent par exemple permettre :
- d'éviter la contamination bactérienne sur des surfaces médicales ou autres ;
- de diminuer la prise de graffiti, c'est-à-dire d'inscriptions, slogans, ou dessins peints, pulvérisés ou gravés sur un mur ou sur une surface et qui ne sont normalement pas prévus à cet effet ;
- de réduire les traces de doigts, notamment sur de l'électroménager ou sur les téléphones portables ou ordinateurs ;
- de faciliter le nettoyage des poêles ;
- de diminuer l'empreinte sur les nouvelles planètes en limitant les contaminations ;
- de réduire les tâches sur les vêtements ;
- de réduire les coûts de nettoyage des avions ;
- d'améliorer la réception d'informations dans les instruments permettant de mesurer la pression subie par l'avion pour en déduire sa vitesse (sondes pitot) ;
- d'augmenter l'imperméabilité spécifique vis-à-vis des liquides des vêtements ;
- d'améliorer l'isolation, notamment des toitures ;
- d'empêcher la contamination biologique, notamment, dans le cas de cibles biologiques, cela peut générer l'antibioadhésion ou inversement de la bioadhésion ou une spécificité de bioadhésion ou d'antibioadhésion en fonction de la morphologie de surface ;
- de protéger les peintures urbaines de la pollution ;
- de réduire l'entretien des systèmes optiques, notamment les lentilles des télescopes, les lunettes, les microscopes ;
- d'éviter le nettoyage des vitres ;
- de réduire les coûts d'entretien et la perte de luminosité sur les panneaux photovoltaïques ;
- d'améliorer la visibilité sur les parebrises ;

Parmi les applications d'antifriction envisageables selon l'invention, les matériaux selon l'invention peuvent par exemple permettre :
- de diminuer la consommation des bateaux ;
- d'améliorer la manoeuvrabilité des bateaux ;
- d'améliorer le comportement à la glisse des sports et jeux de glisse tels notamment ski, snowboard, luge ;
- d'améliorer la performance des nageurs et plongeurs ;
- de réduire la consommation de carburant tel que le kérosène ;
- d'augmenter l'écoulement d'un fluide sur une surface ;
- de diminuer le givre sur les pâles, notamment d'hélicoptère ;
- d'améliorer l'efficacité des turbines ;
- de réduire la formation de givre sur les avions ;
- de réduire la formation de givre sur les câbles ou les échangeurs thermiques et
- d'améliorer le rendement des moteurs.

Parmi les applications d'anticorrosion envisageables selon l'invention, les matériaux selon l'invention peuvent par exemple permettre :
- d'améliorer la protection contre la corrosion des coques de bateaux et tous les matériaux immergés comme les sondes, les bouées, les arbres de transmission et hélices;
- d'augmenter la résistance à l'eau des composants électroniques ; et
- d'améliorer la durée de vie des composants électroniques.

Bien entendu, l'invention ne se limite pas aux modes de réalisations décrits et représentés dans les figures jointes et l'homme du métier pourra être amené, grâce à des opérations de routine, à réaliser d'autres modes de réalisation non décrits explicitement, sans sortir du cadre et de la portée de la présente invention.

### Exemple 1 : Exemple de réalisation d'un revêtement multicouche superhydrophobe :

Pour cet exemple, le Demandeur a développé des revêtements superhydrophobes comprenant deux couches :
- du polypyrrole à titre de promoteur d'adhésion, et
- un polymère fluoré superhydrophobe, qui est le PEdot-F4.

Le PEdot-F4 est un polymère comprenant des monomères Edot-F4 répondant à la formulé générale suivante :

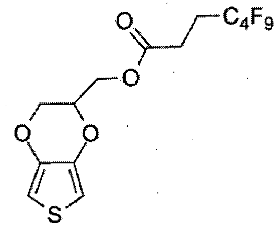

Les deux polymères ci-dessus sont déposés par électrodéposition par chronoampérométrie (à potentiel imposé). Le potentiel d'oxydation est déterminé au préalable par voltamétrie cyclique à 0,1 V/s, de 0 V à 2V.

Dans la cellule électrochimique, la contre-électrode est une plaque de platine et l'électrode de référence est une électrode au calomel saturé (SCE pour « *saturated calomel electrode* »). La solution d'électrolyte est dégazée avant l'expérience.

Le dépôt de polypyrrole a été réalisé dans des solvants différents (acétonitrile et eau). Plusieurs électrolytes peuvent être utilisés :
- le perchlorate de lithium, et
- l'acide sulfurique dans l'acétonitrile
- l'acide fluorosulfonique, et
- l'acide oxalique (dihydrate) dans de l'eau.

Le film de polypyrrole est d'environ 20 nanomètres ou dizaines de nanomètres d'épaisseur.

Le polypyrrole est déposé dans de l'eau ultra pure (R> 18,2MΩ.cm) en utilisant l'acide oxalique dihydrate (57mM) comme électrolyte. L'électrode de travail utilisée est une plaque en acier inoxydable 316.

La charge de polypyrrole déposée est de 5mC/cm² à un potentiel d'oxydation de 0,75V ± 0,03V vs SCE.

Après dépôt, la surface est lavée avec de l'eau ultra pure et séchée.

Le monomère Edot-F4, dont la formule générale est reprise ci-dessus, est électrodéposé sur le film de polypyrrole. La polymérisation est effectuée dans l'électrolyte composé d'acétonitrile anhydre et de perchlorate de lithium ou de Tetrabutylammonium hexafluorophosphate. L'électrode de travail est l'acier inoxydable 316 recouverte de 5mC/cm² de polypyrrole. La charge déposée de PEdot-F4 est de 10mC/cm² à un potentiel d'oxydation de 1,43V ± 0,05V vs SCE.

Après dépôt, la surface est lavée avec de l'acétonitrile et séchée.

L'angle de contact de l'eau du revêtement tel que préparé est d'environ 160°.

Un test d'adhésion a ensuite été réalisé, selon la méthode ASTM D 3359.

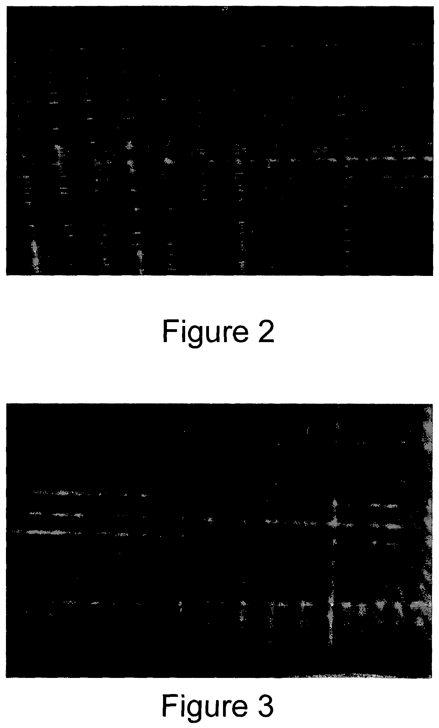

Le résultat de ce test est reproduit aux figures 2 et 3. Il démontre que les adhésions du revêtement de polypyrrole et du revêtement superhydrophobe avec du polypyrrole sur de l'acier inoxydable 316 sont excellentes. Les bords des coupes sont totalement lisses et aucune des cases de la grille ne se détache.

### Exemple 2 : préparation de matériau superhydrophobe selon l'invention et tests de superhydrophobicité et d'adhésion associés.

Pour cet exemple, seize matériaux différents ont été réalisés, pour lesquels l'angle de contact à l'eau a été calculé. Un test d'adhésion a également été réalisé pour chaque matériau, selon la méthode ASTM D 3359.

Avant tout traitement, des surfaces métalliques, qui constituent le substrat conducteur du matériau, ont été nettoyées aux ultra-sons pendant 15 min dans de l'éthanol puis ont été rincées dans de l'éthanol et séchées. Les surfaces métalliques utilisées sont de type acier, mais des dépôts sur du platine, donnent des résultats sensiblement identiques (données non détaillées ci-dessous). D'autres surfaces telles que l'aluminium permettent également le dépôt de pyrrole.

Ensuite, sur le substrat, des traitements par plasma oxygène ou plasma argon, un greffage de monocouche auto-assemblée SAM et/ou une ou deux électrodépositions ont été réalisées.

Le détail des différents modes opératoires de ces étapes sont détaillés ci-dessous :

### a. Plasma 02 :

Après nettoyage, les surfaces d'acier sont traitées par plasma d'oxygène sous un flux de 10sccm à 300W pendant 1 minute. L'électrodéposition du polymère superhydrophobe est réalisée immédiatement après ce traitement.

### b. Plasma Ar :

Après nettoyage, les surfaces d'acier sont traitées par plasma d'argon sous un flux de 10sccm à 300W pendant 1 minute. L'électrodéposition du polymère superhydrophobe est réalisée immédiatement après ce traitement.

### c. Aqua Regia :

Après nettoyage, le substrat est immergé 10 minutes dans une solution d'*Aqua Regia* (acide chlorhydrique/acide nitrique dans les proportions 3:1), puis il est rincé à l'eau déminéralisée et séché.

### d. Monocouche auto-assemblée SAM :

Les molécules greffées sont des acides comme par exemple, l'acide undecylenique, l'acide décanoïque, l'acide linoléique ou l'octadecyl phosphonique acide ou le 11-(1H-pyrrol-1-yl)undecane-1-thiol.

Après nettoyage, les surfaces d'acier sont immergées dans une solution à 2,5mM dans l'éthanol absolu pendant 12h. Les surfaces sont ensuite rincées à l'éthanol absolu, puis séchées.

### e. Electrodéposition Pyrrole Acétonitrile/LiClO4 :

La polymérisation électrochimique du pyrrole s'effectue dans une solution contenant 0,01M de monomère avec du perchlorate de lithium (0,1M) comme électrolyte.

Le polypyrrole est une couche polymérique conductrice dont l'épaisseur doit préférentiellement être inférieure à 0,1 micromètres. L'épaisseur idéale étant comprise entre 1 et 50 nanomètres. Plus préférentiellement encore, l'épaisseur idéale est d'environ 20 nm.

Le solvant utilisé est l'acétonitrile anhydre.

La charge surfacique déposée est d'environ 5mC/cm² avec un potentiel de travail de 1,5V à 1,9V selon la présence d'un traitement préalable.

La contre électrode est une plaque de platine et l'électrode de référence est une électrode au calomel saturée (SCE).

Après déposition la surface est rincée dans le solvant.

### f. Electrodéposition Pyrrole Eau/H2SO4 :

La polymérisation électrochimique du pyrrole s'effectue dans une solution contenant 0,01 M de monomère avec de l'acide sulfurique (0,1M) comme électrolyte. Le solvant utilisé est l'eau ultra pure.

La charge surfacique déposée est de 5mC/cm² avec un potentiel de travail d'environ 1V.

La contre électrode est une plaque de platine et l'électrode de référence est une électrode au calomel saturée (SCE).

Après déposition la surface est rincée dans le solvant.

### g. Electrodéposition Pyrrole Eau/Acide paratoluène-sulfonique :

La polymérisation électrochimique du pyrrole s'effectue dans une solution contenant 0,25 M de monomère avec de l'acide paratoluènesulfonique (1M) comme électrolyte. Le solvant utilisé est l'eau ultra pure.

La charge surfacique déposée est de 20mC/cm² avec un potentiel de travail d'environ 0,70V.

La contre électrode est une plaque de platine et l'électrode de référence est une électrode au calomel saturée (SCE).

Après déposition la surface est rincée dans le solvant.

### h. Electrodéposition copolymère Pyrrole/Aniline Eau/Acide oxalique :

La polymérisation électrochimique du copolymère Pyrrole/Aniline s'effectue dans une solution contenant 0,1 M de chaque monomère avec de l'acide oxalique dihydrate (0,25 M) comme électrolyte. Le solvant utilisé est l'eau ultra pure.

La charge surfacique déposée est de 7mC/cm² avec un potentiel de travail d'environ 1,05V.

La contre électrode est une plaque de platine et l'électrode de référence est une électrode au calomel saturée (SCE).

Après déposition la surface est rincée dans le solvant.

### i. Electrodéposition Pyrrole acétonitrile/HFSO3

La polymérisation électrochimique du pyrrole s'effectue dans une solution contenant 0,01 M de monomère avec de l'acide fluorosulfonique (0,01M) comme électrolyte. Le solvant utilisé est l'acétonitrile anhydre.

La charge surfacique déposée est de 5mC/cm2 avec un potentiel de travail d'environ 1,6V.

La contre électrode est une plaque de platine et l'électrode de référence est une électrode au calomel saturée (SCE).

Après déposition la surface est rincée dans le solvant.

### j. Electrodéposition Pyrrole Eau/Acide oxalique

La polymérisation électrochimique du pyrrole s'effectue dans une solution contenant 0,25M de monomère avec de l'acide oxalique dihydrate (0,057M) comme électrolyte. Le solvant utilisé est l'eau ultra pure.

La charge surfacique déposée est de 5mC/cm2 avec un potentiel de travail d'environ 0,75V.

La contre électrode est une plaque de platine et l'électrode de référence est une électrode au calomel saturée (SCE).

Après déposition la surface est rincée dans le solvant.

### k. Electrodéposition polymère superhydrophobe Acétonitrile/LiClO4 :

La polymérisation électrochimique des polymères superhydrophobe s'effectue dans une solution contenant 0,01M de monomère avec du perchlorate de lithium (0,1M) comme électrolyte.

Les polymères superhydrophobes utilisés sont les suivants :

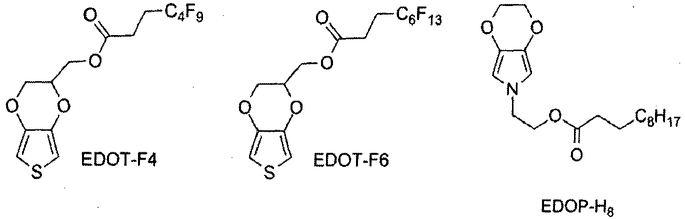

Le solvant utilisé est l'acétonitrile anhydre.

La charge surfacique déposée est d'environ 5mC/cm² avec un potentiel de travail de 1,5V à 1,9V selon la présence d'un traitement préalable.

La contre électrode est une plaque de platine et l'électrode de référence est une électrode au calomel saturée (SCE).

Après déposition la surface est rincée dans le solvant.

Le détail des différents constituants des seize matériaux réalisés est repris dans le tableau 1 ci-dessous. Le tableau 1 reprend également les résultats des mesures de l'angle de contact à l'eau (WCA pour « Water Contact Angle ») ainsi que les résultats du test d'adhésion selon la méthode ASTM D 3359. Il est a noté que les résultats montrant de faibles adhésions (résultat 0B) présentés dans le tableau 1, correspondent à des couches copolymériques dont le dépôt est homogène et permettant de retrouver les propriétés intrinsèques des matériaux superoléophobes et/ou superhydrophobes. Cela est donc une amélioration par rapport aux résultats obtenus pour les dépôts sans traitement de surface ou utilisation de promoteur observés précédemment sur la figure 1.

**Tableau 1 :**

| **Substrat** | **Plasma ou Etching chimique** | **SAM** | **Electrodéposition 1** | **Electrodéposition 2 (polymère SH)** | **WCA** | **Tests adhésion selon norme ASTM D 3359** |
|---|---|---|---|---|---|---|
| Acier | | Acide undecylenique | | Edot-F6 100mC Acetonitrile/ LiClO4 | 153° | 0B |
| Acier | | Octadecyl phosphonique acide | | Edot-F6 100mC Acetonitrile/ LiClO4 | 153° | 0B |
| Acier | | | Pyrrole 10mC Acétonitrile /LiClO4 | Edot-F6 100mC Acetonitrile/ LiClO4 | 151° | 0B |
| Acier | Plasma O2 | | Pyrrole 10mC Acétonitrile /LiClO4 | Edot-F6 100mC Acetonitrile/ LiClO4 | 150° | 0B |
| Acier | Plasma Ar | | Pyrrole 10mC Acétonitrile /LiClO4 | Edot-F6 100mC Acetonitrile/ LiClO4 | 151° | 0B |
| Acier | | Octadecyl phosphonique acide | Pyrrole 10mC Acétonitrile /LiClO4 | Edot-F6 100mC Acetonitrile/ LiClO4 | 150° | 0B |
| Acier | Plasma O2 | Acide décanoïque | Pyrrole 10mC Acétonitrile /LiClO4 | Edot-F6 100mC Acetonitrile/ LiClO4 | 154° | 0B |
| Acier | Plasma O2 | Acide undecylenique | Pyrrole 10mC Acétonitrile /LiClO4 | Edot-F6 100mC Acetonitrile/ LiClO4 | 150° | 0B |
| Acier | Plasma O2 | Octadecyl phosphonique acide | Pyrrole 10mC Acétonitrile /LiClO4 | Edot-F6 100mC Acetonitrile/ LiClO4 | 154° | 0B |
| Acier | | | Pyrrole 10mC Eau/H2SO4 | Edot-F6 100mC Acetonitrile/ LiClO4 | 150° | 0B |
| Acier | | | Pyrrole 10mC Eau/HFSO3 | Edot-F6 100mC Acetonitrile/ LiClO4 | 155° | 5B |
| Acier | | | Pyrrole 5mC Eau/Acide oxalique | Edot-F4 10mC Acétonitrile/ LiClO4 | 160° | 5B |
| Acier | | Dopamine | | Edop-H8 200mC Acétonitrile/ Bu4NPF6 | 150° | 5B |
| Acier | | | Pyrrole 5mC Eau/Acide paratoluenes ulfonique | Edot-F4 20mC Acétonitrile/Li ClO4 | 157 | 5B |
| Acier | Aqua Regia | | Pyrrole 10mC Acétonitrile /LiClO4 | Edot-F4 20mC Acétonitrile/Li ClO4 | 155 | 0B |
| Acier | | | Copolymère Pyrrole /Aniline 7mC eau/Acide oxalique | Edot-F4 25mC Acétonitrile/Li ClO4 | 158 | 0B |

### Exemple 3 : Exemple de préparation de monomères à courte chaine fluorée selon l'invention et étude des propriétés associées. (exemple de référence)

Pour cet exemple, afin d'élaborer des surfaces superoléophobes, des monomères EDOT-NH-Fₙ répondant à la formule générale suivante ont été synthétisés et caractérisés:

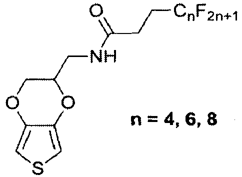

### a. Synthèse des composés

Le schéma de synthèse de ces composés, en trois étapes, est repris ci-dessous :

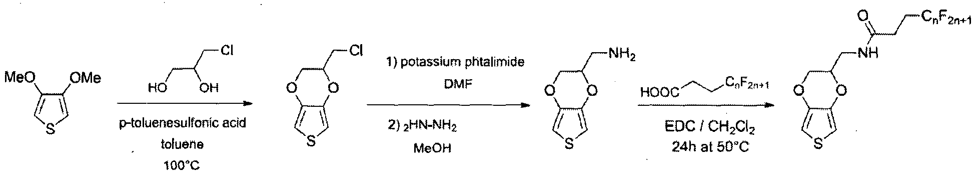

La première étape de la synthèse est la transetherification du 3,4-dimethoxythiophene avec le 3-chloropropane-1,2-diol et permet d'obtenir le 2-(chloromethyl)-2,3-dihydrothieno[3,4-*b*] [1,4]dioxine.

La deuxième étape est la substitution du groupement chloride par une amine primaire en utilisant la réaction de Gabriel en présence de phtalamide de potassium.

Du 2-(chloromethyl)-2,3-dihydrothieno[3,4-*b*][1,4] dioxine (0,3 g, 1,6 mmol) et du phtalamide de potassium (0,4 g, 2,2 mmol) sont ajoutés à 5 mL de DMF. Le mélange est chauffé à 100°C pendant 24 heures.

Ensuite, la solution est versée dans 100 mL d'eau et le produit est extrait avec du chloroforme.

La couche organique est ensuite lavée avec de l'hydroxyde de sodium froid et de l'eau, puis est séchée sur du sodium sulfate Na₂SO₄.

Le produit obtenu et l'hydrate d'hydrazine (0,16 g, 3,2 mmol) sont alors ajoutés à 10 mL de méthanol, le mélange est chauffé à 50°C pendant 1 heure. 25 mL d'eau sont ajoutés et le méthanol est extrait sous vide.

2 mL de HCl concentré sont ensuite ajouté lentement et la solution est chauffée à 60°C pendant 1 heure.

Après filtration, le filtrat est neutralisé avec 2N d'hydroxyde de sodium. Après évaporation du solvant, le produit est purifié par chromatographie sur colonne (gel de silice, éluant: méthanol / dichlorométhane 1:4).

Pour ces expériences, trois chaines fluorées courtes, C₄F₉, C₆F₁₃ et C₈F₁₇, ont été introduites afin de démontrer la possibilité de réaliser des surfaces superoléophobes avec des chaines fluorées courtes.

Les composés suivants ont été synthétisés :
- EDOT-NH2: (2,3-dihydrothieno[3,4-b][1,4]dioxin-2-yl)methanamine ;
- EDOT-NH-F8: N-((2,3-dihydrothieno[3,4-b][1,4]dioxin-2-yl)methyl)-4,4,5,5,6,6,7,7,8,8,9,9,10,10,11,11,11-heptadecafluoroundecan-amide ;
- EDOT-NH-F6: N-((2,3-dihydrothieno[3,4-b][1,4]dioxin-2-yl)methyl)-4,4,5,5,6,6,7,7,8,8,9,9,9-tri-decafluorononanamide ; et
- EDOT-NH-F4: N-((2,3-dihydrothieno[3,4-b][1,4]dioxin-2-yl)methyl)-4,4,5,5,6,6,7,7,7-nonafluoro-heptanamide.

Le Demandeur a pu mettre en évidence que des monomères à chaine fluorée courte peuvent, sous certaines conditions, démontrer des propriétés de répulsion liquide supérieures aux composés à longue chaine fluorée.

### b. Propriétés de surfaces des composés :

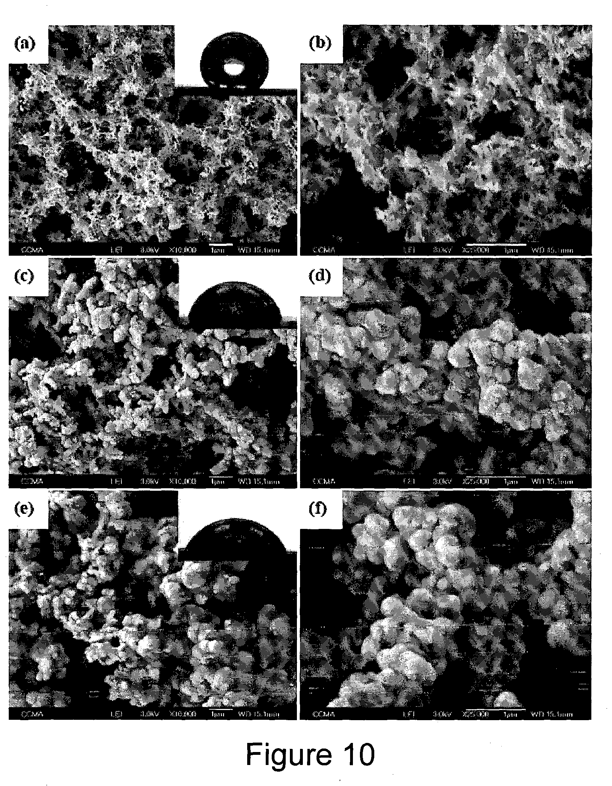

Une charge de dépôt (Qs) de 100 mC/cm² a été utilisée pour le dépôt. Les analyses au microscope électronique à balayage des films polymères ont révélé que la longueur de la chaîne fluorée avait une influence majeure sur la morphologie de surface. Cela est notamment illustré à la figure 10.

L'augmentation de la longueur de la chaîne fluorée induit un changement de morphologie de la surface. On observe un assemblage 3D en forme de fibrilles minces pour les composés C4F9 (figure 10a-b), tandis que les composés C8F17 présentent un assemblage 3D en forme de sphères (figures 10e-f) .

Les analyses de mouillabilité de surface ont été réalisées avec quatre différents liquides sondes :
- Eau (γ L = 72,8 mN/m),
- Diiodométhane (γ L = 50,0 mN/m),
- Huile de tournesol (γ L ≈ 31 mN/m) et
- Hexadécane (γ L = 27,6 mN/m).

Les angles de glissement (α) et l'hystérésis (H) ont été déterminés en utilisant la méthode inclinée à goutte avec 6µL de gouttes de liquide. L'angle de glissement correspond à l'inclinaison de surface minimale pour induire le retrait d'une gouttelette d'eau.

Les angles de mouillage rentrant et sortant, et par conséquent l'hystérésis, ont été obtenus juste avant que la goutte d'eau ne roule sur la surface (l'inclinaison de la surface induit une déformation de la goutte).

Au moment du dépôt, des surfaces superhydrophobes présentant des propriétés autonettoyantes ont été élaborés à partir des trois monomères (θₑₐᵤ = 160,4°, Hₑₐᵤ = 1,3° et αₑₐᵤ = 1,2° pour PEDOT-NH-F₄; θₑₐᵤ = 160,5°, Hₑₐᵤ = 4,6° et αₑₐᵤ = 1,2° pour PEDOT-NH-F₆; θₑₐᵤ = 157.0°, Hₑₐᵤ = 8,0° et αₑₐᵤ = 1,4° pour PEDOT-NH-F₈).

De façon surprenante, lorsque la tension de surface de du liquide sonde diminue (de l'eau à l'hexadécane), une forte diminution de la répulsion liquide a été observée, avec une augmentation de la longueur de la chaîne fluorée.

Les propriétés observées contredisent donc ce qui est connu sur les polymères fluorés. En effet, contrairement à ce qui a été réalisé à ce jour, pour la première fois, des surfaces présentant d'excellentes propriétés superoléophobes ont été développées avec succès à l'aide de chaînes fluorées extrêmement courtes (*F*-butyle).

Afin de bien comprendre l'impact de chaque paramètre sur la mouillabilité de la surface, l'influence de la morphologie/rugosité de surface (partie physique) et de l'hydrophobie intrinsèque des polymères (partie chimique) ont été séparés.

Pour calculer ces paramètres, il est nécessaire de produire des surfaces lisses de chaque polymère afin de mesurer leur mouillabilité. Pour cela, des surfaces « lisses » de polymères ont pu être obtenus en réduisant la charge de dépôt à environ 0,5 mC/cm². La régularité de la surface a été confirmée par profilométrie optique.

Quelle que soit le liquide sonde, une diminution de l'hydro- et d'oléophobie a été observée avec la longueur de la chaîne fluorée.

Les résultats de ces travaux sont repris dans le tableau 2 ci-dessous :

**Tableau 2 : Propriétés hydrofuges pour les polymères « lisses »; Qs = 0,5 mC/cm²**

| | Angles de contact statiques [deg] | | | |
|---|---|---|---|---|
| | Eau | Diiodométhane | Huile de tournesol | Hexadécane |
| PEDOT-NH-F₄ | 93,3 | 62,6 | 51,9 | 43,4 |
| PEDOT-NH-F₆ | 102,5 | 68,3 | 60,3 | 49,1 |
| PEDOT-NH-F₈ | 113,7 | 79,7 | 65,7 | 54,0 |

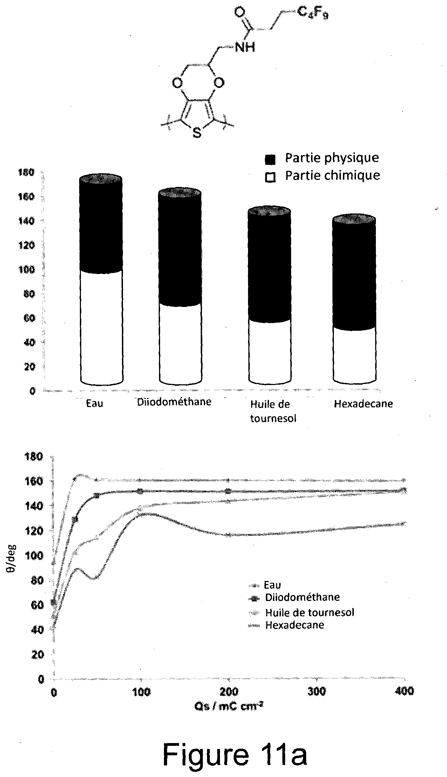

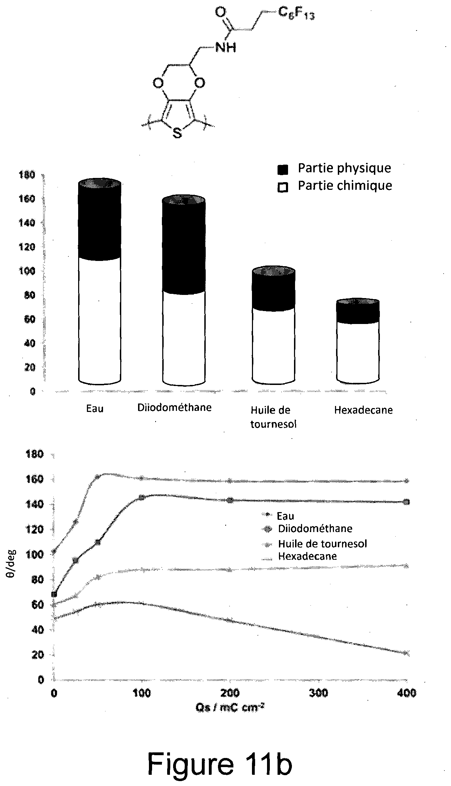

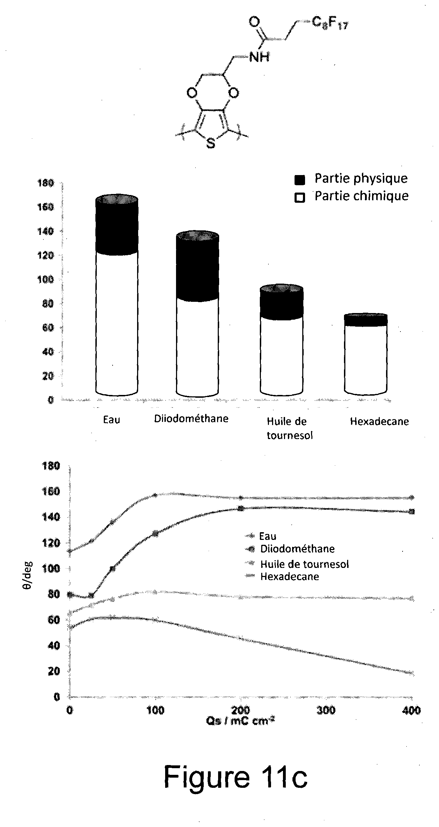

Afin de comprendre l'importance de la rugosité de surface sur les propriétés de répulsion liquide, des dépôts ont été effectués en faisant varier la charge de dépôt (Qs) de 0 à 400 mC/cm². Les figures 11(a), 11(b) et 11(c) montrent l'influence de Qs sur les propriétés répulsives liquides. Comme cela est illustré aux figures 11(a), 11(b) et 11(c), l'augmentation de l'angle de contact n'est pas directement fonction de la charge de dépôt (Qs). La rugosité de surface en revanche est fonction de la charge de dépôt (Qs).

Les trois polymères sont intrinsèquement hydrophobe (θₑₐᵤ polymères « lisses » > 90°), ce qui explique la facilité à produire des surfaces superhydrophobes à partir de ces monomères, tel que décrit par les théories de Wenzel et Cassie-Baxter.

Pour obtenir des propriétés superhydrophobes, des charges de dépôt Qs de 25, 50 et 100 mC/cm², ont été nécessaires pour atteindre les meilleures propriétés d'hydrofugation avec PEDOT-NH-F4, PEDOT-NH-F6 et PEDOT-NH-F8. De telles charges de dépôt correspondent à une rugosité de surface d'environ 0,10 µm, 0,15 µm et 2,2 µm respectivement. Ces analyses confirment la plus grande capacité à produire des surfaces superhydrophobes à l'aide EDOT-NH-F4 en tant que monomère.

De façon intéressante, il apparait que l'angle de contact pour le diiodométhane pour les trois polymères « lisses » est largement inférieur à 90°, alors que des polymères structurés pourraient repousser ce liquide avec un angle de contact supérieur à 140°. Cela dépend de la charge de dépôt comme le montre les figures 11 (a) à (c) (pour Qs ≈ 50 mC/cm² avec EDOT-NH-F4, 100 mC/cm² avec EDOT-NH-F6 et 200 mC/cm² avec EDOT-NH-F8).

Seul le PEDOT-NH-F4 a réussi à repousser l'huile de tournesol avec un angle de contact maximal de 150° lorsque le Qs est de 400 mC/cm², alors que l'angle de contact du PEDOT-NH-F4 « lisse » n'est que de 51,9°, comme cela est décrit au tableau 2 ci-dessus.

Pour les deux autres polymères, l'angle de contact pour l'huile de tournesol des polymères structurés est inférieur à 90° mais supérieur à celui des polymères « lisses » correspondants, dont les valeurs sont reprises dans le tableau 2 ci-dessus.

Pour ce qui concerne l'hexadécane, seul le PEDOT-NH-F4 est capable de repousser ce liquide. Toutefois, il a été observé une forte dépendance de l'angle de contact avec la charge de dépôt Qs. En effet, en raison de la tension superficielle extrêmement faible du liquide sonde, celui-ci est extrêmement sensible au profil de rugosité en raison de sa forte capacité à pénétrer à l'intérieur de cavités extrêmement petites. De façon atypique, un angle de contact maximal de 132° a été mesuré lorsque Qs = 100 mC/cm². Pour PEDOT-NH-F6 et PEDOT-NH-F8, l'angle de contact des films structurés est supérieur à celui des polymères « lisses » correspondants à Qs bas (faible rugosité), mais devient plus bas à Qs élevé (forte rugosité).

L'angle de contact des polymères « lisses », la rugosité de surface et la tension de surface du liquide sonde sont des paramètres importants, mais ne suffisent pas à prédire la possibilité de produire des surfaces superoléophobes.

Les paramètres géométriques caractérisant la morphologie de surface tels que la forme des structures et leurs dimensions sont des paramètres plus importants que le pouvoir de répulsion liquide des polymères.

Dans cet exemple, il apparait que les fibrilles obtenues avec les chaines C₄F₉ induisent la formation de structures plus réentrantes ou à géométrie négative que les structures en chou-fleur obtenues avec des chaines C8F17.

### c. Parties physiques et chimiques des angles de contact :

La détermination des angles de contact sur des surfaces « lisses » de polymères a permis de séparer la contribution en pourcentage de la physique de surface (rugosité de surface/morphologie) et de la contribution en pourcentage de la chimie de surface, sur la valeur de l'angle de contact.

Les calculs ont été réalisés en prenant en considération les résultats obtenus lorsque Qs = 100 mC/cm².

Les résultats sont repris aux figures 11 (a) à (c).

La contribution chimique des polymères fluorés diminue avec le γL du liquide sonde. Elle augmente lorsque la taille de la chaine fluorée augmente.

Pour les polymères fluorés PEDOT-NH-F₆ et PEDOT-NH-F₈, il a été observé que la contribution physique augmente avec le γL de l'eau au diiodométhane, puis diminue du diiodométhane à l'hexadécane. Avec l'hexadécane, la contribution physique représente environ 10% de l'angle de contact pour PEDOT-NH-F₈ et environ 20% pour PEDOT-NH-F₆.

Pour PEDOT-NH-F₄, la contribution physique augmente avec le γL de l'eau à l'hexadécane. La contribution physique représente environ 42% de la valeur de l'angle de contact pour l'eau et 67% de la valeur de l'angle de contact pour l'hexadécane.

Si la contribution physique représente souvent moins de 50% des angles de contact pour les surfaces superhydrophobes, elle devrait être toujours supérieure à 50% pour les surfaces superoléophobiques. Dans le cas présent, environ 2/3 des propriétés superoléophobes sont dues à la contribution physique, c'est-à-dire la rugosité de surface/morphologie.

### Conclusion :

Dans cet exemple, il a été montré qu'il est possible d'obtenir des surfaces qui soient superoléophobes avec des courtes chaines fluorées de type F-butyle (θₕᵤᵢₗₑ de tournesol = 150,0° et θ_{hexadécane} = 132,1°).

Ceci va à l'encontre des connaissances actuelles concernant les polymères fluorés et semble être en partie dû à la présence de fibrilles minces comme détaillé ci-dessus (*cf*. figure 10 (a)).

Afin d'étudier l'impact de la contribution physique (rugosité de surface/morphologie) et de la contribution chimique (hydro/oléophobie intrinsèque) sur l'angle de contact, des surfaces « lisses » de polymères ont été produites et leurs propriétés analysés.

De tels polymères sont oléophiles (pour *F*-butyle, θ_{huile de tournesol} = 51,9° et θ_{hexadécane} = 43,4°). Ceci signifie que c'est la rugosité de surface et la morphologie qui sont capables de modifier les propriétés de la surface en la faisant passer d'oléophile à superoléophobe.

Ici, il semble également que la présence de connecteurs amides permet de réduire la mobilité des chaines fluorées en augmentant leur cristallinité. Cet effet semble être fortement amplifié par la morphologie de surface et la rugosité. En conséquence, les connecteurs amides induisent la formation d'une barrière d'énergie supplémentaire entre l'état Cassie-Baxter et l'état Wenzel, qui stabilise l'état Cassie-Baxter.

### Exemple 4 : Exemple de surfaces superhydrophobes sans fluor à partir de dérivés de 3,4-propylenedioxythiophene (ProDOT) : (exemple de référence)

### a. Synthèse des composés

Le schéma de synthèse des composés ProDOT-Hₙ est repris ci-dessous :

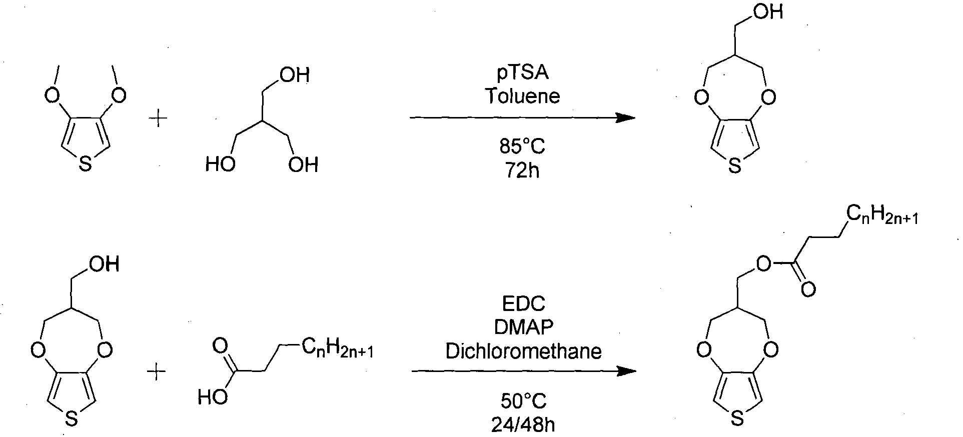

Pour la synthèse des composés, le précurseur hydroxylé ProDOT-OH ((3,4-dihydro-2H-thieno[3,4-b][1,4]dioxepin-3-yl)méthanol a été obtenu par transetherification du 3,4-dimethoxythiophène avec du 2-(hydroxymethyl)-propane-1,3-diol dans du toluène en présence d'une quantité catalytique d'acide p-toluènesulfonique. Ensuite, les monomères originaux (ProDOT-Hₙ pour lesquels n = 4, 6 et 8) ont été obtenus par greffage d'alkyles acides sur du ProDOT-OH par une réaction d'estérification en présence de Chlorhydrate de N-(3-diméthylamino-propyl)-N'-éthylcarbodiimide (EDC) et de (diméthylamino) pyridine (DMAP) dans du dichlorométhane.

Les monomères sont ensuite purifiés par chromatographie sur colonne (gel de silice, éluant: dichlorométhane/cyclohexane 9:1).

La formule générale des composés obtenus est la suivante :

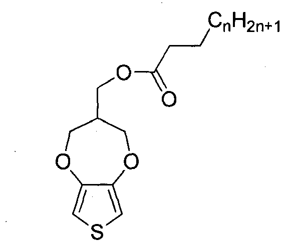

Les composés synthétisés pour cet exemple sont les suivants :
- ProDOT-H₈ : (3,4-dihydro-2H-thieno[3,4-b][1,4]diox-epin-3-yl)methyl undecanoate ;
- ProDOT-H₆ : (3,4-dihydro-2H-thieno[3,4-b][1,4]diox-epin-3-yl)methyl nonanoate ; et
- ProDOT-H₄ : (3,4-dihydro-2H-thieno[3,4-b][1,4]diox-epin-3-yl)methyl heptanoate.

L'électropolymérisation des monomères a été étudiée en utilisant deux solvants différents (l'acétonitrile et le dichlorométhane). Toutes les expériences ont été réalisées avec 0,1 M d'hexafluorophosphate de tétrabutylammonium et 0,01 M de monomère dans une cellule à trois électrodes.

L'électrode de travail est soit un disque de platine permettant d'étudier les paramètres électrochimiques des monomères et polymères, soit une plaque en or permettant d'étudier les propriétés des films polymères électrolytiques. La contre-électrode est une barre de carbone vitreux, et l'électrode de référence est une électrode au calomel saturé.

Tout d'abord, le potentiel d'oxydation du monomère a été mesuré par une méthode de voltampérométrie cyclique rapide (1 cycle de 0 à 2V à 0,1 Vs⁻¹). Ensuite, pour étudier l'aptitude à la polymérisation des films, une voltampérométrie cyclique plus faible est effectuée: dix balayages de -1V à un potentiel légèrement plus faible que le potentiel d'oxydation du monomère à 0,02 Vs⁻¹.

La même expérience de voltampérométrie cyclique est également effectuée dans une solution exempte de monomère, afin de vérifier la stabilité du polymère.

Afin d'étudier les caractéristiques des surfaces de polymères électrodéposées, une plaque d'or d'environ 1 cm² remplace le disque de platine comme électrode de travail. Les films sont déposés à l'aide d'une méthode de chronoampérométrie avec différentes charges de dépôt: 25, 50, 100, 200 et 300 mC/cm².

La mouillabilité de surface a été déterminée en mesurant les angles de contact statiques (CA) sur l'eau (CAₑₐᵤ ; γL = 72,8 mN/m).

La morphologie des surfaces a été étudiée par microscopie électronique à balayage (MEB) et la rugosité a été évaluée par profilométrie optique.

### b. Electrochimie :

Les potentiels d'oxydation des différents monomères, listés dans le tableau 3 ci-dessous, sont plus élevés dans le dichlorométhane que dans l'acétonitrile. La capacité d'électropolymérisation est plus élevée dans l'acétonitrile que dans du dichlorométhane:

**Tableau 3: Potentiel d'oxydation des monomères**

| **Polymère** | **E^{αx} dans l'aoétonitrile [V vs. SCE]** | **E^{αx} dans le dichlorométhane [V vs. SCE]** |
|---|---|---|
| ProDOT-H₈ | 1,56 | 1,91 |
| ProDOT-H₆ | 1,59 | 1,91 |
| ProDOT-H₄ | 1,58 | 1,91 |

L'influence de la nature ou de la longueur des substituants hydrophobes est négligeable.

Les films polymères ont ensuite été électrodéposés par voltampérométrie cyclique (10 cycles) afin d'étudier la croissance et la stabilité des films.

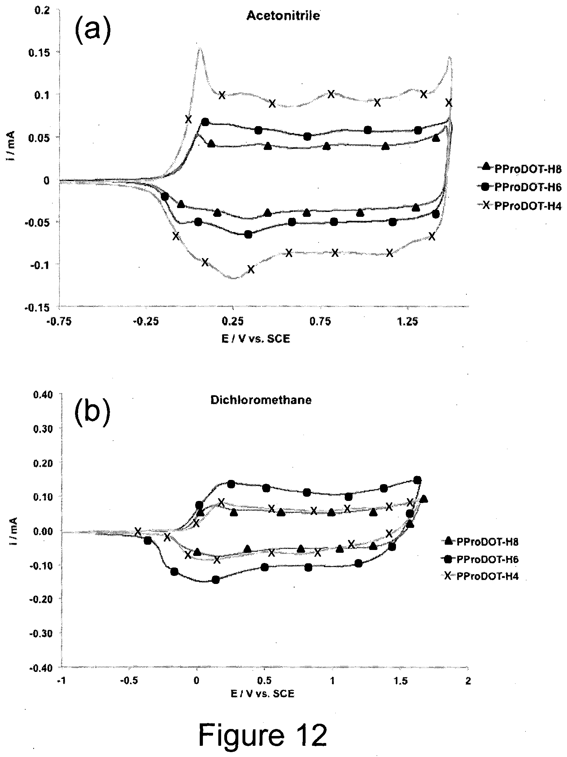

La figure 12 montre les Voltamogrammes cycliques obtenus pour les polymères avec des pics d'oxydation et de réduction.

Chaque polymère s'est parfaitement agrandi à la fois dans l'acétonitrile et dans le dichlorométhane, de façon constante après chaque cycle.

Afin d'étudier leur stabilité, l'électrode de travail a été mise dans une solution sans monomère et dix cycles ont été effectués afin de visualiser s'il y a ou non une perte de signal. Les PProDOT-Hn sont relativement stables dans l'acétonitrile. En revanche, ils sont moins stables dans le dichlorométhane car une fraction de polymère s'est décrochée après chaque cycle.

### c. Mesures de mouillage de surface :

Différentes mesures de mouillage ont été réalisées pour les polymères, dans des solvants différents. Les résultats de ces tests sont repris dans le tableau 4 ci-dessous.

**Tableau 4 : Propriétés hydrofuges des polymères PProDOT; Qs = 100 mC/cm2**

| Polymère | Solvant | CAₑₐᵤ / ° | Hₑₐᵤ / ° | αₑₐᵤ / ° |
|---|---|---|---|---|
| PProDOT-H₈ | Acetonitrile | 132.1 | - | |
| | Dichloromethane | 130.7 | - | |
| PProDOT-H₆ | Acetonitrile | **155.0** | Comportement collant | |
| | Dichloromethane | 129.3 | - | |
| PProDOT-H₄ | Acetonitrile | 117.4 | - | |
| | Dichloromethane | 118.6 | - | |

Le tableau 4 ci-dessus démontre que les polymères PProDOT-H8 et PProDOT-H4 sont hydrophobes, tandis que le polymère PProDOT-H6 est, quant à lui, superhydrophobe, avec un comportement collant. Ceci signifie qu'une goutte d'eau reste attachée à la surface, même si la surface est perturbée.
Le solvant d'électropolymérisation Dichlorométhane a uniquement impacté le polymère PProDOT-H₆, alors que, pour les autres dérivés alkyles PProDOT aucun changement significatif de l'angle de contact à l'eau n'est observée.

Autrement dit, l'électropolymérisation dans le dichlorométhane ne fait pas varier la mouillabilité à l'eau des polymères PproDOt-H4 et PProDOT-H8 mais a un impact significatif sur le PProDOT-H6 (diminution de 25.7°).

### d. Morphologie de la surface.

Afin de mieux comprendre les valeurs d'angle de contact, la morphologie de surface a été étudiée par microscopie électronique à balayage (MEB ou SEM pour Scanning Electron Microscopy en anglais).

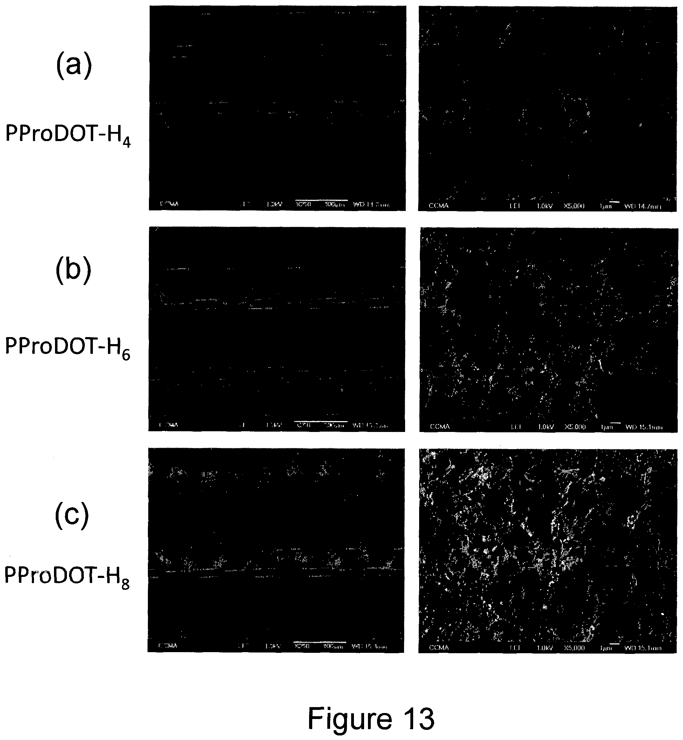

La figure 13 reprend les images MEB des surfaces de polymères PProDOT-Hn.

Le polymère PProDOT-H6 électrodéposé dans de l'acétonitrile est composé d'agglomérats ou de fibres sur un tapis de nanofibres plus petites dont le diamètre est d'environ 100 nm. L'espace entre les agrégats semble être à l'origine du comportement collant de la surface polymérique PProDOT-H6.

A partir de ces images, on peut conclure que le dichlorométhane semble influencer la morphologie de surface des dérivés hydrocarbonés PProDOT. Ces polymères conduisent à des surfaces plus ou moins structurées dans l'acétonitrile où, comme dans le dichlorométhane, ils sont lisses avec de petits trous qui couvrent toute leur surface, quelle que soit la longueur de la chaîne alkyle.

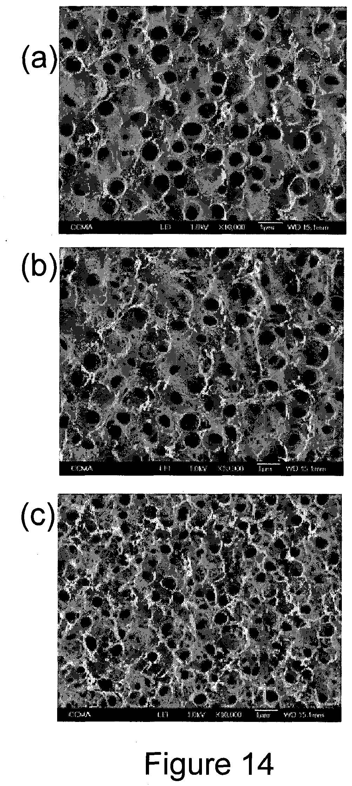

Comme cela est illustré aux figures 14 (a), (b) et (c), le diamètre des trous augmente lorsque la chaîne hydrocarbonée diminue. Pour le PProDOT-H₈, les trous ont un diamètre d'environ 400 nm, pour PProDOT-H6, il est autour de 550 nm, et pour le PProDOT-H4, le diamètre est d'environ 600 nm.

Par conséquent, si l'effet du solvant a déjà été précédemment identifié comme étant un facteur majeur dans l'électrodéposition de dérivés EDOT contenant des chaînes hydrocarbonées (surfaces structurées et lisses obtenues dans l'acétonitrile et le dichlorométhane, respectivement), cet exemple confirme que c'est également le cas pour les expériences réalisées.

En effet, il a été démontré que le solvant influence la solubilité des oligomères formés dans les premiers instants. Dans ces conditions électrochimiques, il a été montré que la taille des pores est contrôlée par la longueur de la chaîne hydrocarbonée greffée sur le coeur polymérisable ProDOT.

## Revendications

1. Matériau superoléophobe et/ou superhydrophobe multicouche comprenant :
d'une part un premier constituant qui est un substrat conducteur ou préalablement rendu conducteur (1) :
• dont la surface est modifiée par traitement chimique et/ou physique (2) et intégrant une première couche conductrice promoteur d'adhésion (3) ; ou
• intégrant une première couche conductrice promoteur d'adhésion (3) ;
et d'autre part au moins un autre constituant qui est une couche polymérique ou copolymérique superoléophobe et/ou superhydrophobe (4, 5 ou 6) composée d'un ou plusieurs monomères à base de cycle aromatique ou hétéroaromatique substitués par une ou plusieurs chaines fluorocarbonées et/ou hydrocarbonées, **caractérisé en ce que** les différents constituants dudit matériau respectent un gradient croissant d'hydrophobie entre la première couche déposée sur le substrat conducteur ou préalablement rendu conducteur (1) et la dernière couche dudit matériau.

2. Matériau selon la revendication 1, **caractérisé en ce que** le substrat conducteur ou préalablement rendu conducteur (1) est choisi parmi de l'acier inoxydable ou non inoxydable, du platine, de l'or, de l'argent, du fer, de l'oxyde d'indium et d'étain ITO, du titane, du carbone vitreux, du silicium, de l'aluminium, du cuivre, du zinc, du nickel, du laiton et du , bronze.

3. Matériau selon la revendication 1, **caractérisé en ce que** le traitement chimique est réalisé par polissage, sablage, brossage et/ou par une attaque chimique par l'eau royale *Aqua regia.*

4. Matériau selon la revendication 1, **caractérisé en ce que** le traitement physique est réalisé par plasma oxygène ou plasma argon.

5. Matériau selon l'une des revendications 1 à 4, **caractérisé en ce que** la première couche conductrice promoteur d'adhésion (3) est déposée selon plusieurs techniques : monocouche auto-assemblée, plasma, sol-gel, électrofilature, lithographie, polymérisation radicalaire, d'électrodéposition, couche par couche, trempage), enduction centrifuge.

6. Matériau selon la revendication 5, **caractérisé en ce que** la monocouche auto-assemblée comprend de l'acide undecylenique, de l'acide undécanoïque, de l'acide décanoïque, de l'acide linoléique, de l'acide octadécyle phosphonique, du pyrrole undécane thiol, du N-(3,4-dihydroxyphenethyl)thiophene-3-carboxamide, de la dopamine, pris seuls ou en mélange.

7. Matériau selon la revendication 5, **caractérisé en ce que** la couche d'électrodéposition est composée d'un ou plusieurs monomères à base de cycle aromatique ou hétéroaromatique éventuellement substitué.

8. Matériau selon la revendication 7, **caractérisé en ce que** la couche d'électrodéposition est composée d'un ou plusieurs monomères de pyrrole ou d'aniline, seuls ou en mélange.

9. Matériau selon l'une des revendications 5 à 8, **caractérisé en ce que** l'épaisseur de la couche conductrice promoteur d'adhésion (3) est inférieure à 100nm.

10. Matériau selon la revendication 9, **caractérisé en ce que** l'épaisseur de la couche conductrice promoteur d'adhésion (3) est de 20nm.

11. Matériau selon l'une des revendications précédentes, **caractérisé en ce que** la couche polymérique ou copolymérique superoléophobe et/ou superhydrophobe (4, 5 ou 6) est composée d'un ou plusieurs monomères répondant à la formule générale (I) suivante : 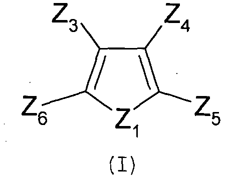 dans laquelle :
• Z₁ est choisi parmi les atomes et groupements S, O, NH et N-W ;
• Z₃ est choisi parmi les atomes et groupements : H, X₁-W, X₁-R₁-X₂, C(R₂)(R₃) ;
• Z₄ est choisi parmi les groupements: W, X₁-W, X₁-R₁-X₂ et C(R₂)(R₃) ;
• Z₅ et Z₆ représentent un atome H ou une chaîne hydrocarbonée comprenant 3 atomes de carbone ;
dans laquelle :
o W représente (CF₂)_{q}F, B₁-(CY₂)ₚY, Q₁-Z₂ ou B₁-Q₁-Z₂ ;
• avec B₁, B₂, B₃ identiques ou différents sont choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S(A₁) ;
pour lequel A₁ et A₂, identiques ou différents représentent :
-B₂-(CY₂)ₚY,
-B₃-ph-Q₂-B₂-(CY₂)ₚY ; « ph » désignant le groupe fonctionnel phényle ;
• avec q compris entre 1 et 20 et p compris entre 0 et 20 ;
• avec Y correspondant à H ou F, sous réserve que, lorsque Y est un atome H, alors p est égal à 0 ;
• avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S, NH ;
∘ R₁ est choisi parmi les groupements -(CH)W-, -(CH₂)ₙ₁-CH(W)- ;-CH(W)-(CH₂)ₙ₁ ; -(CH₂)ₙ₂-CH(W)-(CH₂)n₃- ; et
-(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
• lorsque Z₃ et Z₄ correspondent à X₁-R₁-X₂, ils sont reliés ensemble pour former un cycle ;
• lorsque Z₃ et/ou Z₄ correspondent à C(R₂)(R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
pris seuls ou en mélange.

12. Matériau selon la revendication 11, **caractérisé en ce que** la couche polymérique ou copolymérique superoléophobe et/ou superhydrophobe (4, 5 ou 6) est composée d'un ou plusieurs monomères répondant à la formule générale (I) suivante :
dans laquelle :
• Z₁ est choisi parmi les atomes et groupements S, NH et N-W ;
• Z₃ est choisi parmi les atomes et groupements : H, X₁-W et X₁-R₁-X₂ ;
• Z₄ est choisi parmi les groupements: W, X₁-W et X₁-R₁-X₂ ;
• Z₅ et Z₆ représentent un atome H ;
dans laquelle :
∘ W représente (CF₂)_{q}F, B₁-(CY₂)ₚY, Q₁-Z₂ ou B₁-Q₁-Z₂ ;
• avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S(A₁) ;
pour lequel A₁ et A₂, identiques ou différents représentent :
-B₂-(CY₂)ₚY,
-B₃-ph-Q₂-B₂-(CY₂)ₚY ; « ph » désignant le groupe fonctionnel phényle ;
• avec q compris entre 1 et 16 et p compris entre 0 et 16 ;
• avec Y correspondant à H ou F, sous réserve que, lorsque Y est un atome H, alors p est égal à 0 ;
• avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S;
∘ R₁ est choisi parmi les groupements-(CH)W-, -(CH₂)ₙ₁-CH(W)-,-CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
• lorsque Z₃ et Z₄ correspondent à X₁-R₁-X₂, ils sont reliés ensemble pour former un cycle ;
pris seuls ou en mélange.

13. Matériau selon la revendication 12, **caractérisé en ce que** la couche polymérique ou copolymérique superoléophobe et/ou superhydrophobe (4, 5 ou 6) est composée d'un ou plusieurs monomères choisis parmi:
(i) les composés de formule (I) dans laquelle :
• Z₁ est choisi parmi les atomes et groupements S, NH et N-W ;
• Z₃ est choisi parmi les atomes et groupements H, X₁-W et X₁-R₁-X₂ ;
• Z₄ est choisi parmi les groupements: W, X₁-W et X₁-R₁-X₂ ;
• Z₅ et Z₆ représentent un atome H ;
dans laquelle :
∘ W représente (CF₂)_{q}F, B₁-(CF₂)ₚF, Q₁-Z₂ ou B₁-Q₁-Z₂ ;
• avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S(A₁) ;
pour lequel A₁ et A₂, identiques ou différents représentent :
-B₂-(CF₂)ₚF,
-B₃-ph-Q₂-B₂-(CF₂)ₚF ; « ph » désignant le groupe fonctionnel phényle ;
• avec q et p compris entre 7 et 16 ;
• avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S;
∘ R₁ est choisi parmi les groupements -(CH)W-, -(CH₂)ₙ₁-CH(W)-,-CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
∘ lorsque Z₃ et Z₄ correspondent à X₁-R₁-X₂, ils sont reliés ensemble pour former un cycle ;
ou parmi
(ii) les composés sans fluor suivants, répondant également à la formule générale (I) dans laquelle :
• Z₁ est choisi parmi les atomes et groupements S, 0, NH et N-W ;
• Z₃ est choisi parmi les atomes et groupements H, X₁-W, X₁-R₁-X₂, C(R₂)(R₃) ;
• Z₄ est choisi parmi les atomes et groupements W, X₁-W, X₁-R₁-X₂, C(R₂)(R₃) ;
• Z₅ et Z₆ représentent un atome H ou une chaîne hydrocarbonée comprenant 3 atomes de carbone ;
dans laquelle :
∘ W représente B₁-H, Q₁-Z₂ ou B₁-Q₁-Z₂ ;
• avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S(A₁) ;
pour lequel A₁ et A₂, identiques ou différents représentent :
-B₂-H,
-B₃-ph-Q₂-B₂-H ; « ph » désignant le groupe fonctionnel phényle ;
• avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S, NH ;
∘ R₁ est choisi parmi les groupements -(CH)W-, -(CH₂)ₙ₁-CH(W)- ; -CH(W)-(CH₂)ₙ₁; -(CH₂)ₙ₂-CH(W)-(CH₂)ₙ₃- ; et
-(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
• lorsque Z₃ et Z₄ correspondent à X₁-R₁-X₂, ils sont reliés ensemble pour former un cycle ;
• lorsque Z₃ et/ou Z₄ correspondent à C(R₂)(R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
ou encore parmi
(iii) les composés à courte chaine fluorée suivants répondant également à la formule générale (I) dans laquelle :
• Z₁ est choisi parmi les atomes et groupements S, O, NH et N-W ;
• Z₃ est choisi parmi les atomes et groupements H, X₁-W, X₁-R₁-X₂, C(R₂)(R₃) ;
• Z₄ est choisi parmi les atomes et groupements W, X₁-W, X₁-R₁-X₂, C(R₂)(R₃) ;
• Z₅ et Z₆ représentent un atome H ou une chaîne hydrocarbonée comprenant 3 atomes de carbone ;
dans laquelle :
∘ W représente (CF₂)_{q}F, B₁-(CF₂)ₚF, Q₁-Z₂ ou B₁-Q₁-Z₂ ;
• avec B₁, B₂, B₃ identiques ou différents choisis parmi les groupements : C₁-C₂₀ alkyles, C₁-C₂₀ alkényles, C₁-C₂₀ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S(A₁) ;
pour lequel A₁ et A₂, identiques ou différents représentent :
-B₂-(CF₂)ₚF,
-B₃-ph-Q₂-B₂-(CF₂)ₚF ; « ph » désignant le groupe fonctionnel phényle ;
• avec q et p compris entre 1 et 6 ;
• avec Q₁ et Q₂, identiques ou différents qui représentent OC(O), C(O), SC(O), NHC(O) ;
∘ X₁ et X₂ ; identiques ou différents, sont choisis parmi les atomes ou groupements O, S, NH ;
∘ R₁ est choisi parmi les groupements -(CH)W-, -(CH₂)ₙ₁-CH(W)- ; -CH(W)-(CH₂)ₙ₁; -(CH₂)ₙ₂-CH(W)-(CH₂)ₙ₃- ; et
-(CH₂)ₙ₁- lorsque Z₁ équivaut à N-W ; W étant tel que défini ci-dessus et n₁, n₂, n₃, identiques ou différents, sont compris entre 1 et 12, la somme de n₂ + n₃ étant inférieure ou égale à 12 ;
∘ R₂ est une chaîne hydrocarbonée comprenant 3 atomes de carbone ;
∘ R₃ est choisi parmi les groupements W, X₁-W ou X₁-R₁-X₂;
et dans laquelle :
• lorsque Z₃ et Z₄ correspondent à X₁-R₁-X₂, ils sont reliés ensemble pour former un cycle ;
• lorsque Z₃ et/ou Z₄ correspondent à C(R₂) (R₃), ils sont reliés à Z₆ et Z₅, (Z₆ et Z₃) et (Z₅ et Z₄), pour former un cycle aromatique à 6 carbones, et lorsque R₃ correspond à X₁-R₁-X₂ alors Z₃ et Z₄ sont reliés entre eux et forment également un cycle ;
pris seuls ou en mélange.

14. Matériau selon la revendication 13, **caractérisé en ce que** la couche polymérique ou copolymérique superoléophobe et/ou superhydrophobe (4, 5 ou 6) est composée d'un ou plusieurs monomères choisis parmi les composés sans fluor suivants, répondant à la formule générale (I) dans laquelle :
• Z₁ est choisi parmi les atomes et groupements S, NH et N-W;
• Z₃ est choisi parmi les atomes et groupements H, X₁-W, X₁-R₁-X₂;
• Z₄ est choisi parmi les atomes et groupements W, X₁-W, X₁-R₁-X₂;
• Z₅ et Z₆ représentent un atome H ;
dans laquelle :
∘ W représente B₁-H, Q₁-Z₂ ou b₁-Q₁-Z₂;
• avec B₁, B₂, B₃ identiques ou différents choisis parmi les groupements : C₁-C₁₆ alkyles, C₁-C₁₆ alkényles, C₁-C₁₆ alkynyles, C₆-C₁₈ aryles, C₆-C₁₈ hétéroaryles ;
• avec Z₂ correspondant à A₁, CH(A₁)(A₂), CH₂(A₁), NH(A₁), N(A₁)(A₂), O(A₁) ou S (A₁) ;
pour lequel A₁ et A₂, identiques ou différents représentent :
-B₂-H,
-B₃-ph-Q₂-B₂-H ; « ph » désignant le groupe fonctionnel phényle ;
• avec Q₁ et Q₂, identiques ou différents qui représentent OC(O), C(O), SC(O), NHC(O);
∘ X₁ et X₂ ; identiques ou différents, sont choisis parmi les atomes ou groupements O, S;
∘ R₁ est choisi parmi les groupements -(CH)W-, -(CH₂)ₙ₁-CH(W)-, - CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
lorsque Z₃ et Z₄ correspondent à X₁-R₁-X₂, ils sont reliés ensemble pour former un cycle ;
ou parmi les composés à courte chaine fluorée suivants répondant également à la formule générale (I) dans laquelle :
• Z₁ est choisi parmi les atomes et groupements S, NH et N-W ;
• Z₃ est choisi parmi les atomes et groupements H, X₁-W, X₁-R₁-X₂;
• Z₄ est choisi parmi les atomes et groupements W, X₁-W, X₁-R₁-X₂;
• Z₅ et Z₆ représentent un atome H ;
dans laquelle :
∘ W représente (CF₂)₄F, B₁-(CF₂)₄F, Q₁-Z₂ ou B₁-Q₁-Z₂;
• avec B₁, B₂, B₃ identiques ou différents choisis parmi les groupements : C₁-C₁₄ alkyles;
• avec Z₂ correspondant à A₁, CH(A₁)(A₂), CH₂(A₁), NH(A₁), N(A₁)(A₂), O(A₁) ou S(A₁);
pour lequel A₁ et A₂, identiques ou différents représentent :
-B₂-(CF₂)₄F,
-B₃-ph-Q₂-B₂-(CF₂)₄F ; «ph» désignant le groupe fonctionnel phényle ;
• avec Q₁ et Q₂, identiques ou différents qui représentent OC(O), C(O), SC(O), NHC(O);
∘ X₁ et X₂ ; identiques ou différents, sont choisis parmi les atomes ou groupements 0, S ;
∘ R₁ est choisi parmi les groupements -(CH)W-, -(CH₂)ₙ₁-CH(W)-, - CH(W)-(CH₂)ₙ₁, -(CH₂)ₙ₂-CH(W)-(CH₂)ₙ₃-, -(CH₂)ₙ₁- lorsque Z₁ équivaut à N-W ; W étant tel que défini ci-dessus et n₁, n₂, n₃, identiques ou différents, sont compris entre 1 et 4, la somme de n2 + n3 étant inférieure ou égale à 4 ;
et dans laquelle :
lorsque Z₃ et Z₄ correspondent à X₁-R₁-X₂, ils sont reliés ensemble pour former un cycle;
pris seuls ou en mélange.

15. Procédé de fabrication d'un matériau superoléophobe et/ou superhydrophobe selon l'une des revendications précédentes comprenant les étapes de :
- traitement physique et/ou chimique (2) d'un substrat conducteur ou préalablement rendu conducteur (1) et dépôt d'une couche conductrice promoteur d'adhésion (3) sur ledit substrat ; ou dépôt d'une couche conductrice promoteur d'adhésion (3) sur ledit substrat ;
- dépôt sur la couche conductrice promoteur d'adhésion (3) d'au moins une couche polymérique ou copolymérique superoléophobe et/ou superhydrophobe (4, 5 ou 6) composée d'un ou plusieurs monomères à base de cycle aromatique ou hétéroaromatique substitués par une ou plusieurs chaines fluorocarbonées et/ou hydrocarbonées ; et de
- récupération du matériau superoléophobe et/ou superhydrophobe.

16. Utilisation d'un matériau selon l'une des revendications 1 à 14 comme matériau anti-adhésion.

17. Utilisation d'un matériau selon l'une des revendications 1 à 14 comme matériau antifriction.

18. Utilisation d'un matériau selon l'une des revendications 1 à 14 comme matériau anticorrosion.

## Patentansprüche

1. Superoleophobes und/oder superhydrophobes mehrschichtiges Material, umfassend:
einerseits einen ersten Bestandteil, welcher ein leitfähiges oder zuvor leitfähig gemachtes Substrat (1) ist:
• dessen Oberfläche durch chemische und/oder physikalische Behandlung (2) modifiziert ist und eine erste leitfähige haftvermittelnde Schicht (3) beinhaltet;
oder
• das eine erste leitfähige haftvermittelnde Schicht (3) beinhaltet;
und andererseits mindestens einen anderen Bestandteil, welcher eine superoleophobe und/oder superhydrophobe Polymer- oder Copolymerschicht (4, 5 oder 6) ist, die aus einem oder mehreren Monomeren auf Basis eines aromatischen oder heteroaromatischen Rings besteht, die durch eine oder mehrere Fluorkohlenstoff- und/oder Kohlenwasserstoffketten substituiert sind, **dadurch gekennzeichnet, dass** die verschiedenen Bestandteile des Materials einen ansteigenden Hydrophobiegradienten zwischen der ersten Schicht, die auf dem leitfähigen oder zuvor leitfähig gemachten Substrat (1) abgeschieden ist, und der letzten Schicht des Materials einhalten.

2. Material nach Anspruch 1, **dadurch gekennzeichnet, dass** das leitfähige oder zuvor leitfähig gemachte Substrat (1) ausgewählt ist aus Edelstahl oder Nicht-Edelstahl, Platin, Gold, Silber, Eisen, Indium-Zinn-Oxid ITO, Titan, Glaskohlenstoff, Silizium, Aluminium, Kupfer, Zink, Nickel, Messing und Bronze.

3. Material nach Anspruch 1, **dadurch gekennzeichnet, dass** die chemische Behandlung durch Polieren, Sandstrahlen, Bürsten und/oder durch einen chemischen Angriff mit Königswasser *Aqua regia* ausgeführt wird.

4. Material nach Anspruch 1, **dadurch gekennzeichnet, dass** die physikalische Behandlung durch Sauerstoffplasma oder Argonplasma ausgeführt wird.

5. Material nach einem der Ansprüche 1 bis 4, **dadurch gekennzeichnet, dass** die erste leitfähige haftvermittelnde Schicht (3) nach mehreren Techniken abgeschieden ist: selbstassemblierte Monoschicht, Plasma, Sol-Gel, Elektrospinnen, Lithographie, radikalische Polymerisation, Elektroabscheidung, Schicht für Schicht, Tauchbeschichten, Schleuderbeschichten.

6. Material nach Anspruch 5, **dadurch gekennzeichnet, dass** die selbstassemblierte Monoschicht Undecylensäure, Undecansäure, Decansäure, Linolsäure, Octadecylphosphonsäure, Pyrrolundecanthiol, N-(3,4-Dihydroxyphenethyl)thiophen-3-carboxamid, Dopamin, einzeln genommen oder in Mischung umfasst.

7. Material nach Anspruch 5, **dadurch gekennzeichnet, dass** die Elektroabscheidungsschicht aus einem oder mehreren Monomeren auf Basis eines aromatischen oder heteroaromatischen Rings besteht, der gegebenenfalls substituiert ist.

8. Material nach Anspruch 7, **dadurch gekennzeichnet, dass** die Elektroabscheidungsschicht aus einem oder mehreren Pyrrol- oder Anilinmonomeren, einzeln oder in Mischung, besteht.

9. Material nach einem der Ansprüche 5 bis 8, **dadurch gekennzeichnet, dass** die Dicke der leitfähigen haftvermittelnden Schicht (3) kleiner als 100 nm ist.

10. Material nach Anspruch 9, **dadurch gekennzeichnet, dass** die Dicke der leitfähigen haftvermittelnden Schicht (3) 20 nm beträgt.

11. Material nach einem der vorstehenden Ansprüche, **dadurch gekennzeichnet, dass** die superoleophobe und/oder superhydrophobe Polymer- oder Copolymerschicht (4, 5 oder 6) aus einem oder mehreren Monomeren besteht, die der folgenden allgemeinen Formel (I) entsprechen: 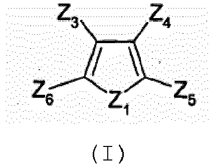 wobei:
• Z₁ ausgewählt ist aus den Atomen und Gruppen S, O, NH und N-W;
• Z₃ ausgewählt ist aus den Atomen und Gruppen: H, X₁-W, X₁-R₁-X₂, C(R₂) (R₃) ;
• Z₄ ausgewählt ist aus den Gruppen: W, X₁-W, X₁-R₁-X₂ und C(R₂)(R₃);
• Z₅ und Z₆ für ein H-Atom oder eine Kohlenwasserstoffkette stehen, die 3 Kohlenstoffatome umfasst;
wobei:
∘ W für (CF₂)_{q}F, B-(CY₂)ₚY, Q₁-Z₂ oder B₁-Q₁-Z₂ steht;
• wobei B₁, B₂, B₃, identisch oder verschieden, ausgewählt sind aus den Gruppen: C₁-C₂₀-Alkyl, C₁-C₂₀-Alkenyl, C₁-C₂₀-Alkinyl, C₆-C₁₈-Aryl, C₆-C₁₈-Heteroaryl;
• wobei Z₂ A₁, CH(A₁)(A₂), CH₂(A₁), NH(A₁), N(A₁)(A₂), O(A₁) oder S(A₁) entspricht;
wobei A₁ und A₂, identisch oder verschieden, stehen für:
-B₂-(CY₂)ₚY,
-B₃-ph-Q₂-B₂-(CY₂)ₚY; wobei "ph" die funktionelle Phenylgruppe bezeichnet;
• wobei q im Bereich zwischen 1 und 20 beträgt und p im Bereich zwischen 0 und 20 beträgt;
• wobei Y H oder F entspricht, unter dem Vorbehalt, dass wenn Y ein H-Atom ist, dann p gleich 0 ist;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S, NH;
∘ R₁ ausgewählt ist aus den Gruppen -(CH)W-, - (CH₂)ₙ₁-CH(W)-; -CH(W)-(CH₂)ₙ₁; -(CH₂)ₙ₂-CH(W)-(CH₂)ₙ₃-; und
-(CH₂)ₙ₁-, wenn Z₁ gleich N-W ist; wobei W so wie oben definiert ist und n₁, n₂, n₃, identisch oder verschieden, im Bereich zwischen 1 und 12 betragen, wobei die Summe aus n₂ + n₃ kleiner als oder gleich 12 ist;
∘ R₂ eine Kohlenwasserstoffkette ist, die 3 Kohlenstoffatome umfasst;
∘ R₃ ausgewählt ist aus den Gruppen W, X₁-W oder X₁-R₁-X₂; und wobei:
• wenn Z₃ und Z₄ X₁-R₁-X₂ entsprechen, dieselben miteinander verbunden sind, um einen Ring zu bilden;
• wenn Z₃ und/oder Z₄ C(R₂) (R₃) entsprechen, dieselben mit Z₆ und Z₅, (Z₆ und Z₃) und (Z₅ und Z₄), verbunden sind, um einen aromatischen Ring mit 6 Kohlenstoffen zu bilden, und wenn R₃ X₁-R₁-X₂ entspricht, dann Z₃ und Z₄ miteinander verbunden sind und ebenfalls einen Ring bilden;
einzeln genommen oder in Mischung.

12. Material nach Anspruch 11, **dadurch gekennzeichnet, dass** die superoleophobe und/oder superhydrophobe Polymer- oder Copolymerschicht (4, 5 oder 6) aus einem oder mehreren Monomeren besteht, die der folgenden allgemeinen Formel (I) entsprechen:
wobei:
• Z₁ ausgewählt ist aus den Atomen und Gruppen S, NH und N-W;
• Z₃ ausgewählt ist aus den Atomen und Gruppen: H, X₁-W und X₁-R₁-X₂;
• Z₄ ausgewählt ist aus den Gruppen: W, X₁-W und X₁-R₁-X₂;
• Z₅ und Z₆ für ein H-Atom stehen;
wobei:
∘ W für (CF₂)_{q}F, B₁-(CY₂)ₚY, Q₁-Z₂ oder B₁-Q₁-Z₂ steht;
• wobei B₁, B₂, B₃, identisch oder verschieden, ausgewählt sind aus den Gruppen: C₁-C₁₆-Alkyl, C₁-C₁₆-Alkenyl, C₁-C₁₆-Alkinyl, C₆-C₁₈-Aryl, C₆-C₁₈-Heteroaryl;
• wobei Z₂A₁, CH(A₁)(A₂), CH₂(A₁), NH(A₁), N(A₁)(A₂), O(A₁) oder S(A₁) entspricht;
wobei A₁ und A₂, identisch oder verschieden, stehen für:
-B₂-(CY₂)ₚY,
-B₃-ph-Q₂-B₂-(CY₂)ₚY; wobei "ph" die funktionelle Phenylgruppe bezeichnet;
• wobei q im Bereich zwischen 1 und 16 beträgt und p im Bereich zwischen 0 und 16 beträgt;
• wobei Y H oder F entspricht, unter dem Vorbehalt, dass wenn Y ein H-Atom ist, dann p gleich 0 ist;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S;
∘ R₁ ausgewählt ist aus den Gruppen -(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁-, wenn Z₁ gleich N-W ist; wobei W so wie oben definiert ist und n₁, n₂, n₃, identisch oder verschieden, im Bereich zwischen 1 und 4 betragen, wobei die Summe aus n2 + n3 kleiner als oder gleich 4 ist;
und wobei:
• wenn Z₃ und Z₄ X₁-R₁-X₂ entsprechen, dieselben miteinander verbunden sind, um einen Ring zu bilden;
einzeln genommen oder in Mischung.

13. Material nach Anspruch 12, **dadurch gekennzeichnet, dass** die superoleophobe und/oder superhydrophobe Polymer- oder Copolymerschicht (4, 5 oder 6) aus einem oder mehreren Monomeren besteht, ausgewählt aus:
(i) den Verbindungen der Formel (I), wobei:
• Z₁ ausgewählt ist aus den Atomen und Gruppen S, NH und N-W;
• Z₃ ausgewählt ist aus den Atomen und Gruppen H, X₁-W und X₁-R₁-X₂;
• Z₄ ausgewählt ist aus den Gruppen: W, X₁-W und X₁-R₁-X₂;
• Z₅ und Z₆ für ein H-Atom stehen;
wobei:
∘ W für (CF₂)_{q}F, B₁-(CF₂)ₚF, Q₁-Z₂ oder B₁-Q₁-Z₂ steht;
• wobei B₁, B₂, B₃, identisch oder verschieden, ausgewählt sind aus den Gruppen: C₁-C₁₆-Alkyl, C₁-C₁₆-Alkenyl, C₁-C₁₆-Alkinyl, C₆-C₁₈-Aryl, C₆-C₁₈-Heteroaryl;
• wobei Z₂ A₁, CH(A₁)(A₂), CH₂(A₁), NH(A₁), N(A₁) (A₂), O(A₁) oder S(A₁) entspricht;
wobei A₁ und A₂, identisch oder verschieden, stehen für:
-B₂- (CF₂)ₚF,
-B₃-ph-Q₂-B₂-(CF₂)ₚF; wobei "ph" die funktionelle Phenylgruppe bezeichnet;
• wobei q und p im Bereich zwischen 7 und 16 betragen;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S;
∘ R₁ ausgewählt ist aus den Gruppen -(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁-, wenn Z₁ gleich N-W ist; wobei W so wie oben definiert ist und n₁, n₂, n₃, identisch oder verschieden, im Bereich zwischen 1 und 4 betragen, wobei die Summe aus n2 + n3 kleiner als oder gleich 4 ist;
und wobei:
∘ wenn Z₃ und Z₄ X₁-R₁-X₂ entsprechen, dieselben miteinander verbunden sind, um einen Ring zu bilden;
oder aus
(ii) den folgenden fluorfreien Verbindungen, die ebenfalls der allgemeinen Formel (I) entsprechen, wobei:
• Z₁ ausgewählt ist aus den Atomen und Gruppen S, O, NH und N-W;
• Z₃ ausgewählt ist aus den Atomen und Gruppen H, X₁-W, X₁-R₁-X₂, C(R₂) (R₃) ;
• Z₄ ausgewählt ist aus den Atomen und Gruppen W, X₁-W, X₁-R₁-X₂, C(R₂) (R₃) ;
• Z₅ und Z₆ für ein H-Atom oder eine Kohlenwasserstoffkette stehen, die 3 Kohlenstoffatome umfasst;
wobei:
∘ W für B₁-H, Q₁-Z₂ oder B₁-Q₁-Z₂ steht;
• wobei B₁, B₂, B₃, identisch oder verschieden, ausgewählt sind aus den Gruppen: C₁-C₂₀-Alkyl, C₁-C₂₀-Alkenyl, C₁-C₂₀-Alkinyl, C₆-C₁₈-Aryl, C₆-C₁₈-Heteroaryl;
• wobei Z₂ A₁, CH(A₁)(A₂), CH₂(A₁), NH(A₁), N(A₁)(A₂), O(A₁) oder S(A₁) entspricht;
wobei A₁ und A₂, identisch oder verschieden, stehen für:
-B₂-H,
-B₃-ph-Q₂-B₂-H; wobei "ph" die funktionelle Phenylgruppe bezeichnet;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S, NH;
∘ R₁ ausgewählt ist aus den Gruppen -(CH)W-, -(CH₂)ₙ₁-CH(W)-; -CH(W)-(CH₂)ₙ₁; -(CH₂)ₙ₂-CH(W)-(CH₂)ₙ₃-; und
-(CH₂)ₙ₁-, wenn Z₁ gleich N-W ist; wobei W so wie oben definiert ist und n₁, n₂, n₃, identisch oder verschieden, im Bereich zwischen 1 und 12 betragen, wobei die Summe aus n₂ + n₃ kleiner als oder gleich 12 ist;
o R₂ eine Kohlenwasserstoffkette ist, die 3 Kohlenstoffatome umfasst;
o R₃ ausgewählt ist aus den Gruppen W, X₁-W oder X₁-R₁-X₂; und wobei:
• wenn Z₃ und Z₄ X₁-R₁-X₂ entsprechen, dieselben miteinander verbunden sind, um einen Ring zu bilden;
• wenn Z₃ und/oder Z₄ C(R₂) (R₃) entsprechen, dieselben mit Z₆ und Z₅, (Z₆ und Z₃) und (Z₅ und Z₄), verbunden sind, um einen aromatischen Ring mit 6 Kohlenstoffen zu bilden, und wenn R₃ X₁-R₁-X₂ entspricht, dann Z₃ und Z₄ miteinander verbunden sind und ebenfalls einen Ring bilden;
oder auch aus
(iii) den folgenden Verbindungen mit kurzer fluorierter Kette, die ebenfalls der allgemeinen Formel (I) entsprechen, wobei:
• Z₁ ausgewählt ist aus den Atomen und Gruppen S, O, NH und N-W;
• Z₃ ausgewählt ist aus den Atomen und Gruppen H, X₁-W, X₁-R₁-X₂, C(R₂) (R₃) ;
• Z₄ ausgewählt ist aus den Atomen und Gruppen W, X₁-W, X₁-R₁-X₂, C(R₂) (R₃) ;
• Z₅ und Z₆ für ein H-Atom oder eine Kohlenwasserstoffkette stehen, die 3 Kohlenstoffatome umfasst;
wobei:
∘ W für (CF₂)_{q}F, B₁-(CF₂)ₚF, Q₁-Z₂ oder B₁-Q₁-Z₂ steht;
• wobei B₁, B₂, B₃, identisch oder verschieden, ausgewählt sind aus den Gruppen: C₁-C₂₀-Alkyl, C₁-C₂₀-Alkenyl, C₁-C₂₀-Alkinyl, C₆-C₁₈-Aryl, C₆-C₁₈-Heteroaryl;
• wobei Z₂ A₁, CH (A₁)(A₂), CH₂(A₁), NH(A₁), N(A₁)(A₂), O(A₁) oder S(A₁) entspricht;
wobei A₁ und A₂, identisch oder verschieden, stehen für:
-B₂- (CF₂)ₚF,
-B₃-ph-Q₂-B₂-(CF₂)ₚF; wobei "ph" die funktionelle Phenylgruppe bezeichnet;
• wobei q und p im Bereich zwischen 1 und 6 betragen;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S, NH;
∘ R₁ ausgewählt ist aus den Gruppen -(CH)W-, -(CH₂)ₙ₁-CH(W)-; -CH(W)-(CH₂)ₙ₁; -(CH₂)ₙ₂-CH(W)-(CH₂)ₙ₃-; und
-(CH₂)ₙ₁-, wenn Z₁ gleich N-W ist; wobei W so wie oben definiert ist und n₁, n₂, n₃, identisch oder verschieden, im Bereich zwischen 1 und 12 betragen, wobei die Summe aus n₂ + n₃ kleiner als oder gleich 12 ist;
∘ R₂ eine Kohlenwasserstoffkette ist, die 3 Kohlenstoffatome umfasst;
∘ R₃ ausgewählt ist aus den Gruppen W, X₁-W oder X₁-R₁-X₂; und wobei:
• wenn Z₃ und Z₄ X₁-R₁-X₂ entsprechen, dieselben miteinander verbunden sind, um einen Ring zu bilden;
• wenn Z₃ und/oder Z₄ C(R₂) (R₃) entsprechen, dieselben mit Z₆ und Z₅, (Z₆ und Z₃) und (Z₅ und Z₄), verbunden sind, um einen aromatischen Ring mit 6 Kohlenstoffen zu bilden, und wenn R₃ X₁-R₁-X₂ entspricht, dann Z₃ und Z₄ miteinander verbunden sind und ebenfalls einen Ring bilden;
einzeln genommen oder in Mischung.

14. Material nach Anspruch 13, **dadurch gekennzeichnet, dass** die superoleophobe und/oder superhydrophobe Polymer- oder Copolymerschicht (4, 5 oder 6) aus einem oder mehreren Monomeren besteht, ausgewählt aus den folgenden fluorfreien Verbindungen, die der allgemeinen Formel (I) entsprechen, wobei:
• Z₁ ausgewählt ist aus den Atomen und Gruppen S, NH und N-W;
• Z₃ ausgewählt ist aus den Atomen und Gruppen H, X₁-W, X₁-R₁-X₂;
• Z₄ ausgewählt ist aus den Atomen und Gruppen W, X₁-W, X₁-R₁-X₂;
• Z₅ und Z₆ für ein H-Atom stehen;
wobei:
∘ W für B₁-H, Q₁-Z₂ oder B₁-Q₁-Z₂ steht;
• wobei B₁, B₂, B₃, identisch oder verschieden, ausgewählt sind aus den Gruppen: C₁-C₁₆-Alkyl, C₁-C₁₆-Alkenyl, C₁-C₁₆-Alkinyl, C₆-C₁₈-Aryl, C₆-C₁₈-Heteroaryl;
• wobei Z₂ A₁, CH(A₁)(A₂), CH₂(A₁), NH(A₁), N(A₁)(A₂), O(A₁) oder S(A₁) entspricht;
wobei A₁ und A₂, identisch oder verschieden, stehen für:
-B₂-H,
-B₃-ph-Q₂-B₂-H; wobei "ph" die funktionelle Phenylgruppe bezeichnet;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S;
∘ R₁ ausgewählt ist aus den Gruppen -(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁-, wenn Z₁ gleich N-W ist; wobei W so wie oben definiert ist und n₁, n₂, n₃, identisch oder verschieden, im Bereich zwischen 1 und 4 betragen, wobei die Summe aus n2 + n3 kleiner als oder gleich 4 ist;
und wobei:
• wenn Z₃ und Z₄ X₁-R₁-X₂ entsprechen, dieselben miteinander verbunden sind, um einen Ring zu bilden;
oder aus den folgenden Verbindungen mit kurzer fluorierter Kette, die ebenfalls der allgemeinen Formel (I) entsprechen, wobei:
• Z₁ ausgewählt ist aus den Atomen und Gruppen S, NH und N-W;
• Z₃ ausgewählt ist aus den Atomen und Gruppen H, X₁-W, X₁-R₁-X₂ ;
• Z₄ ausgewählt ist aus den Atomen und Gruppen W, X₁-W, X₁-R₁-X₂ ;
• Z₅ und Z₆ für ein H-Atom stehen;
wobei:
∘ W für (CF₂)₄F, B₁-(CF₂)₄F, Q₁-Z₂ oder B₁-Q₁-Z₂ steht;
• wobei B₁, B₂, B₃, identisch oder verschieden, ausgewählt sind aus den Gruppen: C₁-C₁₄-Alkyl;
• wobei Z₂ A₁, CH(A₁)(A₂), CH₂(A₁), NH(A₁), N(A₁) (A₂), O(A₁) oder S(A₁) entspricht;
wobei A₁ und A₂, identisch oder verschieden, stehen für: -B₂-(CF₂)₄F,
-B₃-ph-Q₂-B₂-(CF₂)₄F; wobei "ph" die funktionelle Phenylgruppe bezeichnet;
• wobei Q₁ und Q₂, identisch oder verschieden, die für OC(O), C(O), SC(O), NHC(O) stehen;
∘ X₁ und X₂; identisch oder verschieden, ausgewählt sind aus den Atomen oder Gruppen O, S;
∘ R₁ ausgewählt ist aus den Gruppen -(CH)W-, -(CH₂)ₙ₁-CH(W)-, -CH(W)-(CH₂)ₙ₁, -(CH₂)ₙ₂-CH(W)-(CH₂)ₙ₃-, -(CH₂)ₙ₁-, wenn Z₁ gleich N-W ist; wobei W so wie oben definiert ist und n₁, n₂, n₃, identisch oder verschieden, im Bereich zwischen 1 und 4 betragen, wobei die Summe aus n2 + n3 kleiner als oder gleich 4 ist;
und wobei:
wenn Z₃ und Z₄ X₁-R₁-X₂ entsprechen, dieselben miteinander verbunden sind, um einen Ring zu bilden;
einzeln genommen oder in Mischung.

15. Verfahren zur Herstellung eines superoleophoben und/oder superhydrophoben Materials nach einem der vorstehenden Ansprüche, umfassend die Schritte des:
- physikalischen und/oder chemischen Behandelns (2) eines leitfähigen oder zuvor leitfähig gemachten Substrats (1) und Abscheidens einer leitfähigen haftvermittelnden Schicht (3) auf dem Substrat; oder Abscheidens einer leitfähigen haftvermittelnden Schicht (3) auf dem Substrat;
- Abscheidens, auf der leitfähigen haftvermittelnden Schicht (3), von mindestens einer superoleophoben und/oder superhydrophoben Polymer- oder Copolymerschicht (4, 5 oder 6), die aus einem oder mehreren Monomeren auf aromatischer oder heteroaromatischer Ringbasis besteht, die durch eine oder mehrere Fluorkohlenstoff- und/oder Kohlenwasserstoffketten substituiert sind; und des
- Gewinnens des superoleophoben und/oder superhydrophoben Materials.

16. Verwendung eines Materials nach einem der Ansprüche 1 bis 14 als Antihaft-Material.

17. Verwendung eines Materials nach einem der Ansprüche 1 bis 14 als Antifriktionsmaterial.

18. Verwendung eines Materials nach einem der Ansprüche 1 bis 14 als Antikorrosionsmaterial.

## Claims

1. Multilayer superoleophobic and/or superhydrophobic material comprising:
on the one hand, a first constituent that is a conductive substrate or a substrate that has previously been rendered conductive (1):
- the surface of which is modified by chemical and/or physical treatment (2) and that incorporates a first adhesion-promoting conductive layer (3);
or
- that incorporates a first adhesion-promoting conductive layer (3);
and, on the other hand, at least one other constituent that is a superoleophobic and/or superhydrophobic polymer or copolymer layer (4, 5 or 6) composed of one or more monomers based on an aromatic or heteroaromatic ring substituted by one or more fluorocarbon and/or hydrocarbon chains, **characterised in that** the various constituents of said material comply with an increasing hydrophobicity gradient between the first layer deposited on the conductive substrate or substrate previously rendered conductive (1) and the last layer of said material.

2. Material according to claim 1, **characterised in that** the conductive substrate or a substrate that has previously been rendered conductive (1) is chosen from stainless or non-stainless steel, platinum, gold, silver, iron, indium-tin-oxide ITO, titanium, vitreous carbon, silicon, aluminium, copper, zinc, nickel, brass and bronze.

3. Material according to claim 1, **characterised in that** the chemical treatment is carried out by polishing, sandblasting, brushing and/or by a chemical attack via *Aqua regia.*

4. Material according to claim 1, **characterised in that** the physical treatment is carried out via oxygen plasma or argon plasma.

5. Material according to one of claims 1 to 4, **characterised in that** the first adhesion-promoting conductive layer (3) is deposited according to several techniques: self-assembled monolayer, plasma, sol-gel, electrospinning, lithography, radical polymerisation, electrodeposition, layer by layer, dip coating, spin coating.

6. Material according to claim 5, **characterised in that** the self-assembled monolayer comprises undecylenic acid, undecanoic acid, decanoic acid, linoleic acid, octadecylphosphonic acid, pyrrole undecane thiol, N-(3,4-dihydroxyphenethyl)thiophene-3-carboxamide, dopamine, taken alone or in a mixture.

7. Material according to claim 5, **characterised in that** the layer of electrodeposition is comprised of one or more monomers based on an aromatic or heteroaromatic ring which may be substituted.

8. Material according to claim 7, **characterised in that** layer of electrodeposition is comprised of one or more monomers of pyrrole or of aniline, alone or in a mixture.

9. Material according to one of claims 5 to 8, **characterised in that** the thickness of the adhesion-promoting conductive layer (3) is less than 100nm.

10. Material according to claim 9, **characterised in that** the thickness of the adhesion-promoting conductive layer (3) is 20nm.

11. Material according to one of the preceding claims, **characterised in that** the superoleophobic and/or superhydrophobic polymer or copolymer layer (4, 5 or 6) is comprised of one or more monomers having the following general formula (I): 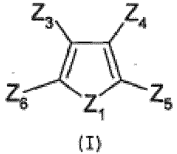 wherein:
- Z₁ is chosen from the atoms and groups S, O, NH and N-W;
- Z₃ is chosen from the atoms and groups: H, X₁-W, X₁-R₁-X₂, C(R₂) (R₃) ;
- Z₄ is chosen from the groups: W, X₁-W, X₁-R₁-X₂ and C(R₂)(R₃) ;
- Z₅ and Z₆ represent a H atom or a hydrocarbon chain comprising 3 carbon atoms;
wherein:
- W represents (CF₂)_{q}F, B₁,-(CY₂)ₚY, Q₁-Z₂ or B₁-Q₁-Z₂;
-- with B₁, B₂, B₃ identical or different are chosen from the groups: C₁-C₂₀ alkyls, C₁-C₂₀ alkenyls, C₁-C₂₀ alkynyls, C₆-C₁₀ aryls, C₆-C₁₈ heteroaryls;
-- with Z₂ corresponding to A₁, CH(A₁)(A₂), CH₂(A₁), NH(A₁), N(A₁)(A₂), O(A₁) or S(A₁) ;
for which A₁ and A₂, identical or different represent: -B₂-(CY₂)ₚY,
-B₃-ph-Q₂-B₂-(CY₂)ₚY; "ph" designating the phenyl functional group;
-- with q between 1 and 20 and p between 0 and 20;
-- with Y corresponding to H or F, provided that, when Y is a H atom, then p is equal to 0;
-- with Q₁ and Q₂, identical or different that represent OC(O), C(O), SC(O), NHC(O);
- X₁ and X₂; identical or different, are chosen from the atoms or groups O, S, NH;
- R₁ is chosen from the groups -(CH)W-, -(CH₂)ₙ₁-CH (W)-; - CH(W)-(CH₂)ₙ₁; -(CH₂)ₙ₂-CH(W)-(CH₂)ₙ₃-; and
- (CH₂)ₙ₁- when Z₁ equivalent to N-W; W being such as defined hereinabove and n₁, n₂ n₃, identical or different, are between 1 and 12, the sum of n2 + n3 being less than or equal to 12;
- R₂ is a hydrocarbon chain comprising 3 carbon atoms;
- R₃ is chosen from the groups W, X₁-W or X₁-R₁-X₂; and
wherein:
- when Z₃ and Z₄ correspond to X₁-R₁-X₂, they are joined together in order to form a ring;
- when Z₃ and/or Z₄ correspond to C(R₂) (R₃), they are joined to Z₆ and Z₅, (Z₆ and Z₃) and (Z₅ and Z₄), in order to form an aromatic ring with 6 carbons, and when R₃ corresponds to X₁-R₁-X₂ then Z₃ and Z₄ are joined together and also form a ring;
taken alone or in a mixture.

12. Material according to claim 11, **characterised in that** the superoleophobic and/or superhydrophobic polymer or copolymer layer (4, 5 or 6) is comprised of one or more monomers having the following general formula (I):
wherein:
- Z₁ is chosen from the atoms and groups S, NH and N-W;
- Z₃ is chosen from the atoms and groups: H, X₁-W and X₁-R₁-X₂;
- Z₄ is chosen from the groups: W, X₁-W and X₁-R₁-X₂;
- Z₅ and Z₆ represent a H atom;
wherein:
- W represents (CF₂)_{q}F, B₁-(CY₂)ₚY, Q₁-Z₂ or B₁-Q₁-Z₂;
-- with B₁, B₂, B₃ identical or different chosen from the groups: C₁-C₁₆ alkyls, C₁-C₁₆ alkenyls, C₁-C₁₆ alkynyls, C₆-C₁₈ aryls, C₆-C₁₈ heteroaryls;
-- with Z₂ corresponding to A₁, CH(A₁)(A₂), CH₂(A₁), NH(A₁), N(A₁)(A₂), O(A₁) or S(A₁) ;
for which A₁ and A₂ identical or different represent: -B₂-(CY₂)ₚY,
-B₃-ph-Q₂-B₂-(CY₂)ₚY; "ph" designating the phenyl functional group;
-- with q between 1 and 16 and p between 0 and 16;
-- with Y corresponding to H or F, provided that, when Y is a H atom, then p is equal to 0;
-- with Q₁ and Q₂, identical or different that represent OC(O), C(O), SC(O), NHC(O);
- X₁ and X₂; identical or different, are chosen from the atoms or groups O, S;
- R₁ is chosen from the groups -(CH)W-, -(CH₂)ₙ₁-CH(W)-, - CH(W)-(CH₂)ₙ₁, -(CH₂)ₙ₂-CH(W)-(CH₂)ₙ₃, -(CH₂)ₙ₁- when Z₁, equivalent to N-W; W being such as defined hereinabove and n₁, n₂, n₃, identical or different, are between 1 and 4, the sum of n2 + n3 being less than or equal to 4;
and wherein:
- when Z₃ and Z₄ correspond to X₁-R₁-X₂, they are joined together in order to form a ring;
taken alone or in a mixture.

13. Material according to claim 12, **characterised in that** the superoleophobic and/or superhydrophobic polymer or copolymer layer (4, 5 or 6) is comprised of one or more monomers chosen from:
(i) the compounds having formula(I) wherein:
- Z₁ is chosen from the atoms and groups S, NH and N-W;
- Z₃ is chosen from the atoms and groups H, X₁-W and X₁-R₁-X₂ ;
- Z₄ is chosen from the groups: W, X1-W and X₁-R₁-X₂;
- Z₅ and Z₆ represent a H atom;
wherein:
- W represents (CF₂)_{q}F, B₁-(CF₂)ₚF, Q₁-Z₂ or B₁-Q₁-Z₂;
-- with B₁, B₂, B₃ identical or different chosen from the groups: C₁-C₁₆ alkyls, C₁-C₁₆ alkenyls, C₁-C₁₆ alkynyls, C₆-C₁₈ aryls, C₆-C₁₈ heteroaryls;
-- with Z₂ corresponding to A₁, CH(A₁)(A₂), CH₂(A₁), NH(A₁), N(A₁)(A₂), O(A₁) or S(A₁);
for which A₁ and A₂, identical or different represent: -B₂- (CF₂)ₚF,
-B₃-ph-Q₂-B₂- (CF₂)ₚF; "ph" designating the phenyl functional group;
-- with q and p between 7 and 16;
-- with Q₁ and Q₂, identical or different that represent OC(O), C(O), SC(O), NHC(O);
- X₁ and X₂; identical or different, are chosen from the atoms or groups O, S;
- R₁ is chosen from the groups -(CH)W-, -(CH₂)ₙ₁-CH(W)-, □CH(W)-(CH₂)ₙ₁, -(CH₂)ₙ2-CH(W)-(CH₂)ₙ₃-, -(CH₂)ₙ₁- when Z₁ equivalent to N-W; W being such as defined hereinabove and n₁ n₂, n₃, identical or different, are between 1 and 4, the sum of n2 + n3 being less than or equal to 4;
and wherein:
- when Z₃ and Z₄ correspond to X₁-R₁-X₂, they are joined together in order to form a ring;
or from
(ii) the following compounds without fluorine, that also have the general formula (I) wherein:
- Z₁ is chosen from the atoms and groups S, O, NH and N-W;
- Z₃ is chosen from the atoms and groups H, X₁-W, X₁-R₁-X₂, C(R₂)(R₃);
- Z₄ is chosen from the atoms and groups W, X₁-W, X₁-R₁-X₂, C(R₂)(R₃);
- Z₅ and Z₆ represent a H atom or a hydrocarbon chain comprising 3 carbon atoms;
wherein:
- W represents B₁-H, Q₁-Z₂ or B₁-Q₁-Z₂;
-- with B₁, B₂, B₃ identical or different chosen from the groups: C₁-C₂₀ alkyls, C₁-C₂₀ alkenyls, C₁-C₂₀ alkynyls, C₆-C₁₈ aryls, C₆-C₁₈ heteroaryls;
-- with Z₂ corresponding to A₁, CH(A₁)(A₂), CH₂(A₁), NH(A₁), N(A₁)(A₂), O(A₁)or S(A₁);
for which A₁ and A₂, identical or different represent: -B₂-H,
-B₃-ph-Q₂-B₂-H; "ph" designating the phenyl functional group;
-- with Q₁ and Q₂, identical or different that represent OC(O), C(O), SC(O), NHC(O);
- X₁ and X₂; identical or different, are chosen from the atoms or groups O, S, NH;
- R₁ is chosen from the groups -(CH)W-, -(CH₂)ₙ₁-CH(W)-;-CH(W)-(CH₂)ₙ₁; -(CH₂)n₂-CH(W)-(CH₂)ₙ₃-; and
- (CH₂)ₙ₁- when Z₁ equivalent to N-W; W being such as defined hereinabove and n₁, n₂, n₃, identical or different, are between 1 and 12, the sum of n₂ + n₃ being less than or equal to 12;
- R₂ is a hydrocarbon chain comprising 3 carbon atoms;
- R₃ is chosen from the groups W, X₁-W or X₁-R₁-X₂; and
wherein:
- when Z₃ and Z₄ correspond to X₁-R₁-X₂, they are joined together in order to form a ring;
- when Z₃ and/or Z₄ correspond to C(R₂)(R₃), they are joined to Z₆ and Z₅, (Z₆ and Z₃) and (Z₅ and Z₄), in order to form an aromatic ring with 6 carbons, and when R₃ corresponds to X₁-R₁-X₂ then Z₃ and Z₄ are joined together and also form a ring;
or from
(iii) the following short-chain fluorine-containing compounds that also have the general formula (I) wherein:
- Z₁ is chosen from the atoms and groups S, O, NH and N-W;
- Z₃ is chosen from the atoms and groups: H, X₁-W, X₁-R₁-X₂, C(R₂)(R₃);
- Z₄ is chosen from the groups: W, X₁-W, X₁-R₁-X₂ and C(R₂)(R₃);
- Z₅ and Z₆ represent a H atom or a hydrocarbon chain comprising 3 carbon atoms;
wherein:
- W represents (CF₂)_{q}F, B₁, -(CF₂)ₚF, Q₁-Z₂ or B₁-Q₁-Z₂;
-- with B₁, B₂, B₃ identical or different are chosen from the groups: C₁-C₂₀ alkyls, C₁-C₂₀ alkenyls, C₁-C₂₀ alkynyls, C₆-C₁₈ aryls, C₆-C₁₈ heteroaryls;
-- with Z₂ corresponding to A₁, CH(A₁)(A₂), CH₂(A₁), NH(A₁), N(A₁)(A₂), O(A₁) or S(A₁);
for which A₁ and A₂ identical or different represent: -B₂-(CF₂)ₚF,
-B₃-ph-Q₂-B₂-(CF₂)ₚF; "ph" designating the phenyl functional group;
-- with q and p between 1 and 6;
-- with Q₁ and Q₂, identical or different that represent OC(O), C(O), SC(O), NHC(O);
- X₁ and X₂; identical or different, are chosen from the atoms or groups O, S, NH;
- R1 is chosen from the groups -(CH)W-, -(CH₂)ₙ₁-CH(W)-;-CH(W)-(CH₂)ₙ₁; -(CH₂)ₙ₂-CH(W)-(CH₂)ₙ₃-; and
- (CH₂)ₙ₁- when Z₁ equivalent to N-W; W being such as defined hereinabove and ₙ₁, n₂, n₃, identical or different, are between 1 and 12, the sum of n₂ + n₃ being less than or equal to 12;
- R₂ is a hydrocarbon chain comprising 3 carbon atoms;
- R₃ is chosen from the groups W, X₁-W or X₁-R₁-X₂: and
wherein:
- when Z₃ and Z₄ correspond to X₁-R₁-X₂, they are joined together in order to form a ring;
- when Z₃ and/or Z₄ correspond to C(R₂)(R₃), they are joined to Z₆ and Z₅, (Z₆ and Z₃) and (Z₅ and Z₄), in order to form an aromatic ring with 6 carbons, and when R₃ corresponds to X₁-R₁-X₂ then Z₃ and Z₄ are joined together and also form a ring;
taken alone or in a mixture.

14. Material according to claim 13, **characterised in that** the superoleophobic and/or superhydrophobic polymer or copolymer layer (4, 5 or 6) is comprised of one or more monomers chosen from the following compounds without fluorine, having the general formula (1) wherein:
- Z₁ is chosen from the atoms and groups S, NH and N-W;
- Z₃ is chosen from the atoms and groups H, X₁-W, X₁-R₁-X₂;
- Z₄ is chosen from the atoms and groups W, X₁-W, X₁-R₁-X₂;
- Z₅ and Z₆ represent a H atom;
wherein:
- W represents B₁-H, Q₁-Z₂ or B₁-Q₁-Z₂;
-- with B₁, B₂ B₃ identical or different chosen from the groups: C₁-C₁₆ alkyls, C₁-C₁₆ alkenyls, C₁-C₁₆ alkynyls, C₆-C₁₈ aryls, C₆-C₁₈ heteroaryls;
-- with Z₂ corresponding to A₁, CH(A₁)(A₂), CH₂(A₁), NH(A₁), N(A₁)(A₂), O(A₁)or S(A₁);
for which A₁ and A₂, identical or different represent: -B₂-H,
-B₃-ph-Q₂-B₂-H; "ph" designating the phenyl functional group;
-- with Q₁ and Q₂, identical or different that represent OC(O), C(O), SC(O), NHC(O);
- X₁ and X₂; identical or different, are chosen from the atoms or groups O, S;
- R₁ is chosen from the groups -(CH)W-, -(CH₂)ₙ₁-CH(W)-, CH(W)-(CH₂)ₙ₁, -(CH₂)ₙ₂-CH(W)-(CH₂)ₙ₃-, - (CH₂)ₙ₁- when Z₁ equivalent to N-W; W being such as defined hereinabove and ₙ₁, n₂, n₃; identical or different, are between 1 and 4, the sum of n2 + n3 being less than or equal to 4;
and wherein:
when Z₃ and Z₄ correspond to X₁-R₁-X₂, they are joined together in order to form a ring;
or from the following short-chain fluorine-containing compounds that also have the general formula (I) wherein:
- Z₁ is chosen from the atoms and groups S, NH and N-W;
- Z₃ is chosen from the atoms and groups H, X₁-W, X₁-R₁-X₂;
- Z₄ is chosen from the atoms and groups W, X₁-W, X₁-R₁-X₂;
- Z₅ and Z₆ represent a H atom;
wherein:
- W represents (CF₂)₄F, B₁-(CF₂)₄F, Q₁-Z₂ or B₁-Q₁-Z₂;
-- with B₁, B₂, B₃ identical or different chosen from the groups: C₁-C₁₄ alkyls;
-- with Z₂ corresponding to A₁, CH(A₁)(A₂), CH₂(A₁), NH(A₁), N(A₁)(A₂), O(A₁) or S(A₁);
for which A₁ and A₂, identical or different represent: -B₂-(CF₂)₄F,
-B₃-ph-Q₂-B₂- (CF₂)₄F; "ph" designating the phenyl functional group;
-- with Q₁ and Q₂, identical or different that represent OC(O), C(O), SC(O), NHC(O);
- X₁ and X₂; identical or different, are chosen from the atoms or groups O, S;
- R₁ is chosen from the groups -(CH)W-, -(CH₂)ₙ₁-CH(W)-,-CH(W)-(CH₂)ₙ₁, -(CH₂)ₙ₂-CH(W)-(CH₂)ₙ₃-, -(CH₂)ₙ₁- when Z₁ equivalent to N-W; W being such as defined hereinabove and n₁, n₂, n₃, identical or different, are between 1 and 4, the sum of n2 + n3 being less than or equal to 4;
and wherein:
when Z₃ and Z₄ correspond to X₁-R₁-X₂, they are joined together in order to form a ring;
taken alone or in a mixture.

15. Method for manufacturing a superoleophobic and/or superhydrophobic material according to one of the preceding claims comprising the steps of:
- physical and/or chemical treatment (2) of a conductive substrate or a substrate that has previously been rendered conductive (1) and deposition of an adhesion-promoting conductive layer (3) on said substrate; or deposition of an adhesion-promoting conductive layer (3) on said substrate;
- deposition on the adhesion-promoting conductive layer (3) of at least one superoleophobic and/or superhydrophobic polymer or copolymer layer (4, 5 or 6) comprised of one or more monomers based on an aromatic or heteroaromatic ring substituted by one or more fluorocarbon and/or hydrocarbon chains; and of
- recovering superoleophobic and/or superhydrophobic material.

16. Use of a material according to one of claims 1 to 14 as an anti-adhesion material.

17. Use of a material according to one of claims 1 to 14 as an anti-friction material.

18. Use of a material according to one of claims 1 to 14 as an anti-corrosion material.
